# EUROPEAN PATENT SPECIFICATION

(11) **EP 2 882 848 B1**
(45) Date of publication and mention of the grant of the patent: **01.11.2017**
(21) Application number: 13827480.8
(22) Date of filing: 07.08.2013
(51) Int. Cl.: C12N 5/071, C12N 5/077

(54) **METHOD, COMBINATION AND/OR COMPOSITION FOR INDUCING CARDIOMYOCYTE DIFFERENTATION**
VERFAHREN, KOMBINATION UND/ODER ZUSAMMENSETZUNG ZUR INDUKTION EINER KARDIOMYOZYTEN-DIFFERENZIERUNG
PROCÉDÉ, COMBINAISON ET/OU COMPOSITION POUR INDUIRE LA DIFFÉRENCIATION DES CARDIOMYOCYTES

(30) Priority: 07.08.2012 US 201261680512 P
(43) Date of publication of application: 17.06.2015
(73) Proprietor: Singapore Health Services Pte Ltd, Singapore 168753 (SG)
(72) Inventor: SHIM, Se Ngie, Winston, Singapore 168753 (SG); MEHTA, Ashish, Singapore 168753 (SG); SEQUIERA, Glen Lester, Singapore 168753 (SG)
(74) Representative: Bailey, Jennifer Ann
(86) International application number: PCT/SG2013/000334
(87) International publication number: WO 2014/025315

(56) References cited:
- WO-A1-2007/070964
- WO-A1-2011/157029
- WO-A1-2012/067266
- AGAPIOS SACHINIDIS ET AL: "Identification of Small Signalling Molecules Promoting Cardiac-Specific Differentiation of Mouse Embryonic Stem Cells", CELLULAR PHYSIOLOGY AND BIOCHEMISTRY, vol. 18, no. 6, 20 November 2006 (2006-11-20), pages 303-314, XP055230342, DOI: 10.1159/000097608
- GRAICHEN R ET AL.: 'Enhanced cardiomyogenesis of human embryonic stem cells by a small molecular inhibitor ofp38 MAPK.' DIFFERENTIATION. vol. 76, no. 4, April 2008, pages 357 - 70, XP026772221
- GAUR M ET AL.: 'Timed inhibition of p38MAPK directs accelerated differentiation of human embryonic stem cells into cardiomyocytes.' CYTOTHERAPY. vol. 12, no. 6, October 2010, pages 807 - 17, XP055196533
- BURRIDGE PW ET AL.: 'Production of de novo cardiomyocytes:human pluripotent stem cell differentiation and direct reprogramming.' CELL STEM CELL. vol. 10, no. 1, 06 January 2012, pages 16 - 28, XP028434719
- MUMMERY CL ET AL.: 'Differentiation of human embryonic stem cells and induced pluripotent stem cells to cardiomyocytes: a methods overview.' CIRC RES. vol. 111, no. 3, 20 July 2012, pages 344 - 58, XP055187227
- VARADARAJAN, S: 'p38 MAPKs coordinately regulate distinct phases of autophagy and lysosomal biogenesis.' PROQUEST 2011, XP055196557
- GAITANAKI C ET AL.: 'et al. Oxidative stress stimulates multiple MAPK signalling pathways and phosphorylation of the small HSP27 in the perfused amphibian heart.' J EXP BIOL. vol. 206, August 2003, pages 2759 - 69., XP055196560
- YAN P ET AL.: 'Cyclosporin-A potently induces highly cardiogenic progenitors from embryonic stem cells.' BIOCHEM BIOPHYS RES COMMUN. vol. 379, no. 1, 30 January 2009, pages 115 - 20, XP025770136
- LI J ET AL.: 'The NADPH oxidase NOX4 drives cardiac differentiation: Role in regulating cardiac transcription factors and MAP kinase activation.' MOL BIOL CELL. vol. 17, no. 9, September 2006, pages 3978 - 88, XP055196563
- BIKKAVILLI RK ET AL.: 'p38 mitogen-activated protein kinase regulates canonical Wnt-beta- catenin signaling by inactivation of GSK3beta.' J CELL SCI. vol. 121, 01 November 2008, pages 3598 - 607, XP055196567
- MA L ET AL.: 'Mitogen-activated protein kinase p38 regulates the Wnt/cyclic GMP/Ca2+ non-canonical pathway.' J BIOL CHEM. vol. 282, no. 39, 28 September 2007, pages 28980 - 90, XP055196569
- SMITH JL ET AL.: 'Targeting combinatorial transcriptional complex assembly at specific modules within the interleukin-2 promoter by the immunosuppressant SB203580.' J BIOL CHEM. vol. 278, no. 42, 17 October 2003, pages 41034 - 46, XP055196570
- James Hudson ET AL: "Primitive Cardiac Cells from Human Embryonic Stem Cells", Stem Cells and Development, vol. 21, no. 9, 10 June 2012 (2012-06-10), pages 1513-1523, XP055157300, ISSN: 1547-3287, DOI: 10.1089/scd.2011.0254

## Description

### Field of the invention

The present invention relates to stem cell technology, In particular, the present invention relates to inducing differentiation of stem cells to cardiomyocytes. These cardiomyocytes have potential for use in therapy.

### Background of the invention

Ischaemic heart disease is one of the most prevalent causes of death and may present with chest pain, myocardial infarction and heart failure. There may be substantial loss of functional cardiomyoctyes with inadequate renewal. While the heart has the regenerative capacity to self-repair the damaged myocardium, this activity is limited and cannot restore complete cardiac function and thus has to be supported by pharmacological interventions. However, these pharmacological interventions currently utilized to manage patients do not restore cardiac function completely. One resort for complete functional restoration is through cardiac transplantation, although this has several limitations such as unavailability of suitable donors, high costs as well as immune-compatibility. The emergence of cell-based therapies and regenerative intervention is a novel approach for treatment of cardiac patients. While adult stem cells (e.g. multipotent cells derived from blood or bone marrow) have been shown to provide some beneficial effects in clinical trials, there is still no consensus on their clinical outcomes.

Human induced pluripotent stem cells (hiPSCs) made from human somatic cells have provided researchers a unique tool for scientific research in the development of cell based regenerative therapeutics and patient disease-modeling. Unlike human embryonic stem cells (hESCs), hiPSCs offers a source of immunocompatible replacement cells that could help in regenerating the scarred myocardium. Studies have also demonstrated that human iPSC, like hESCs have the ability to self-renew and also differentiate into various cell types of the three germ layers (ectoderm, mesoderm and endoderm).

Reprogramming of patient fibroblasts, obtained from skin biopsies with defined transcriptional factors like Oct-4, Sox-2, Klf-4 and c-myc to generate induced pluripotent stem cells (iPSCs) has been demonstrated utilising viral as well as non-viral methods. Early methods used to generate iPSCs required the viral integration of defined vectors in the host genome. Although virus-based reprogramming technology is a robust technique, capable of generating hiPSC with high transfection efficiency, it is also associated with major shortcomings, such as genomic integration, mutation and tumourigenesis. This drawback could impede the downstream application for research and more importantly, limit the possibility for therapeutic purposes. Furthermore, major difficulties include the delivery of reprogramming factors, efficiency and safety. Recent advances have led to the employment of virus-free and vector-free techniques such as episomal vectors, mini-circle, piggyback, lincRNA, microRNA and protein. Non-integrating reprogramming methodologies focus on the generation of safe and genetically unmodified iPSC which can be applied clinically.

In mammalian development, cardiomyocytes are generated from the lateral mesoderm. Cardiomyogenesis could be regulated *in vitro* from hiPSC by a controlled differentiation process, which involves various signaling molecules and extracellular environment. While several approaches are available to differentiate pluripotent stem cells towards cardiomyocytes, high variability in cardiomyogenesis exists with different hiPSC/hESC lines. Inducing cardiomyogenesis in ESCs using various molecules have been reported. For example, the use of SB203580 (a p38-MAPK inhibitor) was shown to enhance cardiomyogenesis in hESCs (Graichen *et al.,* 2008(. IWP-4 (an inhibitor of Wnt signalling) was also reported to initiate differentiation into immature or primitive cardiomyocytes (Hudson *et al.,* 2012). A number of molecules in cardiac-specific differentiation of mouse ESCs were also studied and cyclosporine A identified as having a positive cardiomyogenic effect in mouse ESCs. In order to benefit from the potential of hiPSC-derived cardiomyocytes (hiPSC-CMs) for molecular, structural and functional research, a highly efficient, cost effective and clinically compliant method, which is easily reproducible, is desirable.

### Summary of the invention

Described is a method for inducing cardiomyocyte differentiation comprising the steps of:
(i) providing at least one pluripotent stem cell;
(ii) contacting the pluripotent stem cell(s) with:
   (a) at least one p38 mitogen-activated protein kinase (p38-MAPK) modulator;
   (b) at least one immunomodulator; and
   (c) at least one Wnt modulator.

According to a related aspect, the method comprises the steps of:
(i) providing at least one pluripotent stem cell;
(ii) contacting the pluripotent stem cell(s) with:
   (a) at least one p38 mitogen-activated protein kinase (p38-MAPK) inhibitor;
   (b) at least one immunosuppressant; and
   (c) at least one Wnt modulator.

Accordingly, the invention provides an in vitro method for inducing cardiomyocyte differentiation comprising the steps of:
(i) providing at least one induced pluripotent stem cell or embryoid body comprising induced pluripotent stem cells;
(ii) contacting said pluripotent stem cell(s) or said embryoid body with:
   (a) SB 203580 (4-[4-(4-fluorophenyl)-2-(4-methylsulfinylphenyl)-1H-imidazol-5-yl]pyridine;
   (b) cyclosporin-A (CSA); and
   (c) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) at a final concentration of 5 µM.

The method may comprise additionally contacting the pluripotent stem cell(s) or enbryoid body with at least one Reactive Oxygen Species (ROS) modulator.

A combination and/or kit could be used comprising at least one p38 mitogen-activated protein kinase (p38-MAPK) modulator; at least one immunomodulator; and at least one Wnt modulator. In particular, the combination and/or kit may be for use in inducing cardiomyocyte differentiation. The combination and/or kit may additionally comprise at least one ROS modulator.

A composition comprising at least one p38 mitogen-activated protein kinase (p38-MAPK) modulator; at least one immunomodulator; and at least one Wnt modulator may be provided. In particular, the composition may be for use in inducing cardiomyocyte differentiation. The composition may further comprise at least one ROS modulator.

### Brief description of the figures

Figure 1 illustrates the differentiation protocol used for hESC and hiPSC in Example 1. On day 0, undifferentiated colonies were transferred into EB suspension culture medium for Embryoid Body (EB) formation. After EB formation for 2 days, cells were treated with four agents, alone or in combination with at least one other agent. Cells were plated on day 8, observed and characterised. Final evaluations of beating clusters were performed on day 21.
Figure 2 shows the morphology of (A) Embryoid Bodies (EBs) in suspension and on plating; (B) Neural Rosette; (C) spontaneously beating EB clusters and (D) Cystic EBs.
Figure 3 shows the percentage of beating EBs for hESC (H9) and hiPSC (C2 and C3) after treatment with (A) SB203580 at a final concentration of 5 µM in the culture medium and (B) SB203580 and Cyclosporin-A at a final concentration of 5 µM and 3 µg/ml of the culture medium, respectively (Example 1).
Figure 4 shows the percentage of beating EBs for (A) hiPSC C2 cell line and (B) hiPSC C3 cell line after treatment with SB203580 at a final concentration of 5 µM; Cyclosporin-A at a final concentration of 3 µg/ml; various final concentrations WI(1): 1 µM; WI(2): 3 µM WI(3): 5 µM and WI(4): 7 µM; of Wnt modulator IWP4; in the culture medium (Example 1).
Figure 5 shows the percentage of beating EBs for (A) hiPSC C2 cell line and (B) hiPSC C3 cell line after treatment with SB203580 at a final concentration of 5 µM; Cyclosporin-A at a final concentration of 3 µg/ml; various final concentrations of Wnt modulator IWP4 [see Figure 4 above] and ROS activator (H₂O₂) at a final concentration of 1 nM; in the culture medium (Example 1).
Figure 6 shows the percentage of beating EBs for (A) hiPSC C2 cell line and (B) hiPSC C3 cell line after treatment with SB203580 at a final concentration of 5 µM; Cyclosporin-A at a final concentration of 3 µg/ml; various final concentrations of Wnt modulator IWP4 [see Figure 4 above] and ROS activator (H₂O₂) at a final concentration of 10 nM; in the culture medium (Example 1).
Figure 7 shows the comparison of temporal gene expression levels of early markers of pluripotency, mesodermal and cardiac mesodermal markers of the C2 and C3 cell lines during cardiomyogenic commitment and development (Example 1).
Figure 8 shows the comparison of temporal gene expression levels of intermediate cardiac-associated transcription factors of the C2 and C3 cell lines, during cardiomyogenic commitment and development (Example 1).
Figure 9 shows the comparison of temporal gene expression levels of cardiac-associated structural genes of the C2 and C3 cell lines during cardiomyogenic commitment and development (Example 1).
Figure 10 shows the comparison of temporal gene expression levels of cardiac-associated ion channel-encoding genes of the C2 and C3 cell lines during cardiomyogenic commitment and development (Example 1).
Figure 11 shows standardization of cardiac differentiation (Example 2) with A showing immunostaining of pluripotent markers, Oct-4, Nanog, SSEA4, Tra-1-60 and Tra-1-81 in undifferentiated hiPSC colonies. Scale bar: 200 µm; B showing relative gene expression level of various markers with and without treatment with growth-factor cocktail (S1) during first 4 days of differentiation.
Note the significant upregulation of T, Mesp1 showing mesodermal commitment; C showing relative gene expression levels of cardiac commitment markers along with Wnts from day 4 to 6 in the presence or absence of growth-factor cocktail (S2) and D showing Relative gene expression levels of Nkx2.5 and Wnt 11 in the presence and absence of VEGF. Bars represent mean ± SEM of three independent experiments.

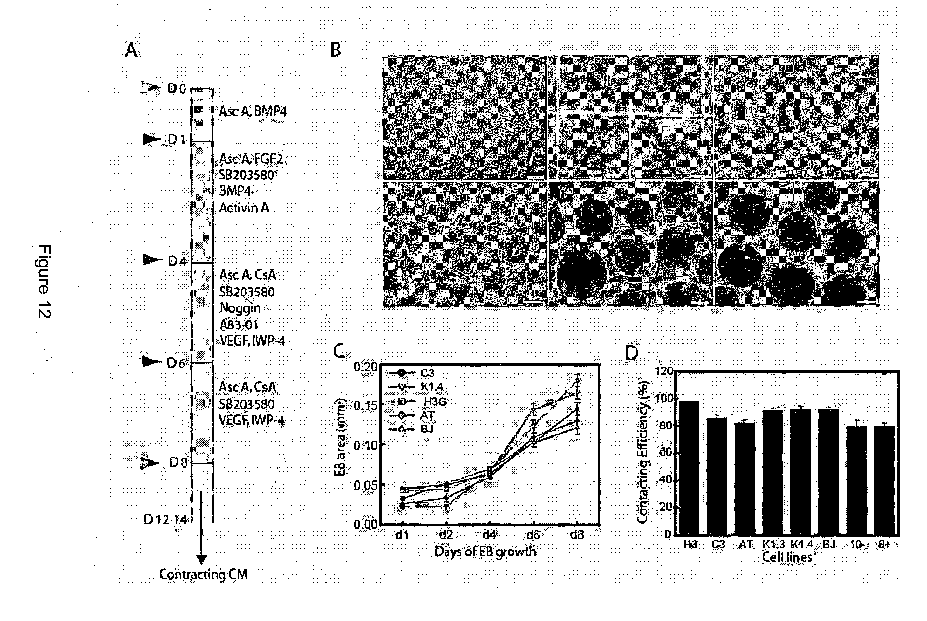

Figure 12 shows the modulation of EBs during cardiac differentiation (Example 2) with A showing a schematic representation of the cardiac differentiation protocol; B being representative images showing changes in EBs during different days of differentiation. i, undifferentiated iPSC; ii-vi, EBs at d1, 2, 4, 6 and 8 of differentiation, respectively. Note the significant increase in the size of EBs during the differentiation Scale bar: 200 µm; C showing a graph representing the increase in EB size during differentiation across multiple hPSC lines. Data represented is a mean ± SEM of three independent experiments and D showing the contracting efficiency post-day 14 following cardiac differentiation across multiple hPSC lines. Bars represent mean ± SEM of three independent experiments.

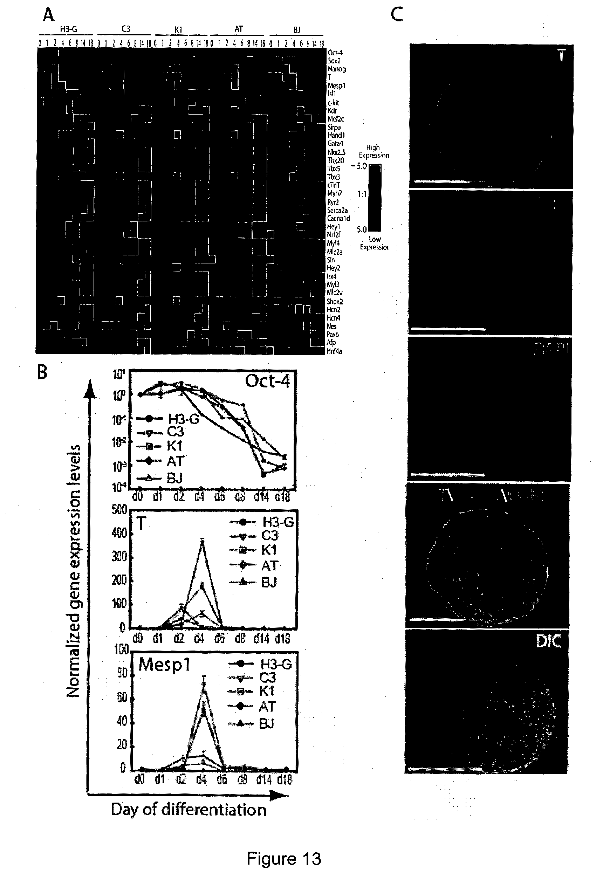

Figure 13 shows the temporal expression during cardiac commitment (Example 2) with A showing a heatmap shows temporal expression of cardiac ontogeny across 5 PSC lines, H3G, C3, K, AT and BJ. Normalized Ct values were plotted, where lower Ct values indicate higher expression (green); B showing temporal gene expression levels of early cardiac commitment markers (T and Mesp1) in multiple cell lines. Note significant up-regulation of these markers by day 4 in all cell lines. Data represented is a mean ± SEM of three independent experiments and C showing whole mount staining of day 4 EBs displaying co-expression of T and Mesp1. Scale bar: 50 µm.

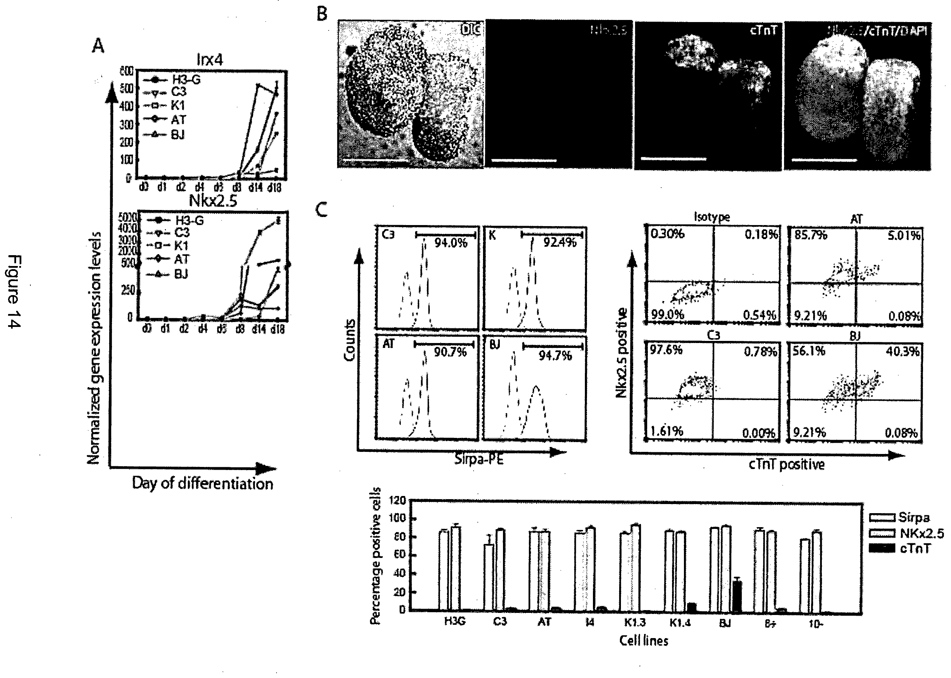

Figure 14 illustrating temporal expression in cardiac progenitors (Example 2) with A showing relative gene expression markers indicative of cardiac progenitors. Note significant increase in the expression of these markers post-day 6 of differentiation. Data represented is a mean ± SEM of three independent experiments; B showing whole mount staining of day 8 EBs displaying co-expression of NKx2.5 and cardiac troponin T. Scale bar: 50 µm and C, top panel: Flow cytometric analysis of day 8 EBs show cells expressing Sirpa (left panel) and NKx2.5/cTnT (right panel) across multiple cell lines. More than 90% of the population express Sirpa and NKx2.5. Only BJ hiPSC cells show significant expression of cTnT on day 8. Bottom panel: Summary of FACS analysis across multiple cell lines at day 8 of differentiation. Data represented is a mean ± SEM of three independent experiments where 10,000 gated events were analysed.

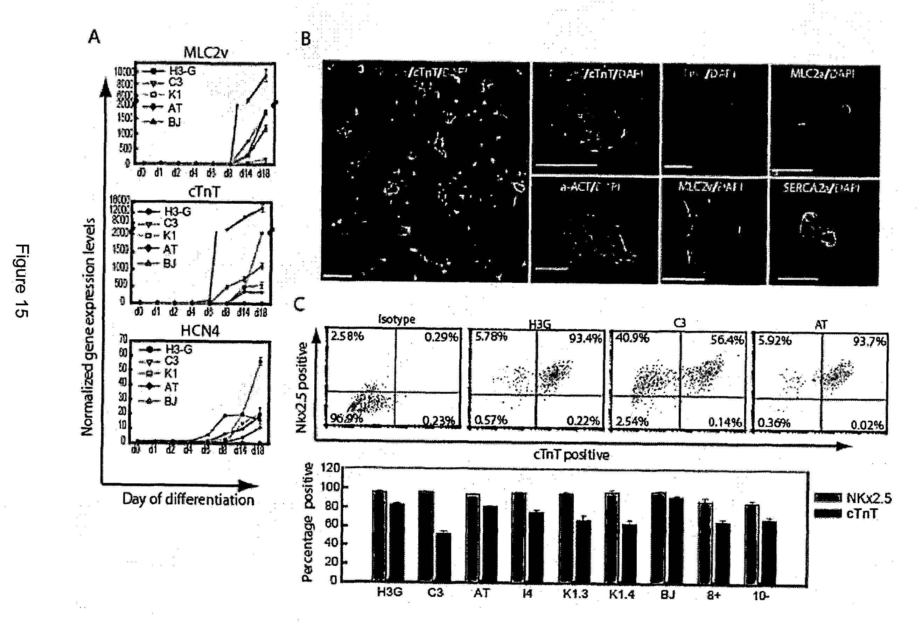

Figure 15 show temporal expression in cardiomyocytes (Example 2) with A showing relative gene expression of markers indicative of cardiomyocyte phenotype. Note the significant increase in the expression levels of these markers post day 8 of differentiation across multiple cell lines. Data represented is a mean ± SEM of three independent experiments; B showing immunostaining of various markers, NKx2.5, cTnT, Titin, MLC2a, α-actinin, MLC2v and SERCA2a confirming cardiac phenotype at day 20. Note the presence of I- and A-bands in these cardiomyoytes and C, Top panel: Flow cytometric analysis of day 18 myocytes shows dual expression of Nkx2.5 and cTnT positive population across multiple cell lines. Bottom panel: Summary of FACS analysis across multiple cell lines at day 18 of differentiation. Data represented is a mean ± SEM of three independent experiments where 10,000 gated events were analysed.

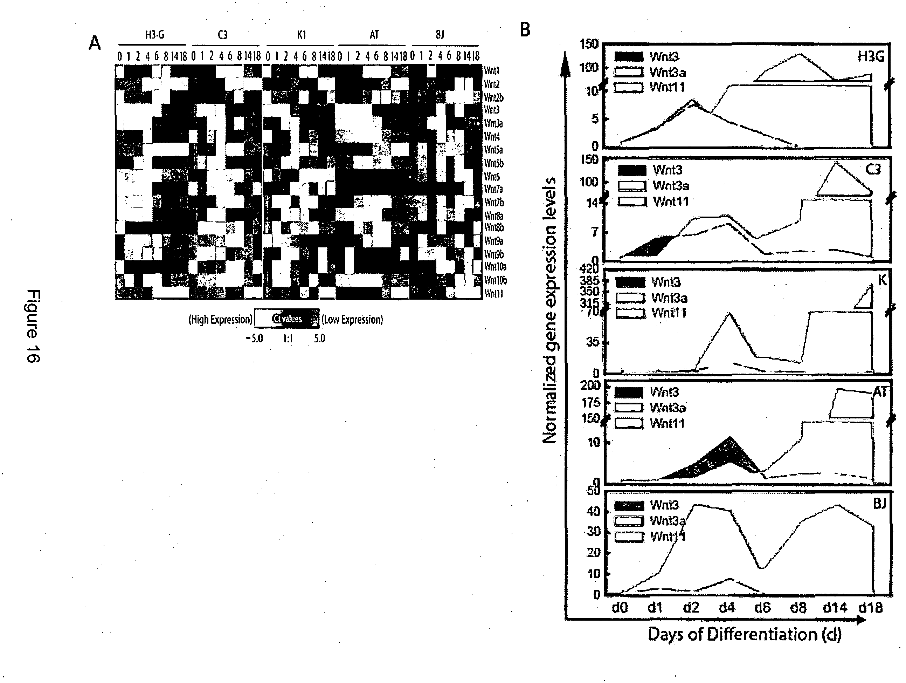

Figure 16 shows the Wnt profile during cardiac differentiation with A showing a heatmap shows temporal expression of Wnts across 5 PSC lines, H3G, C3, K, AT and BJ during cardiac differentiation. Normalized Ct values were plotted, where lower Ct values indicate higher expression (green) and B showing relative gene expression levels of Wnt 3, Wnt 3a and Wnt 11 during cardiac differentiation. Note the significant increase in the Wnt 11 levels post day 6 of differentiation across multiple cell lines.

### Definitions

An "embryoid body" refers to an aggregate of cells derived from pluripotent cells, where cell aggregation can be initiated by any method that prevents the cells from adhering to a surface to form typical colony growth.

The term "induced pluripotent stem cell" refers to a pluripotent stem cell derived from a non-pluripotent cell (e.g. an adult somatic cell). Induced pluripotent stem cells are identical to embryonic stem cells in the ability to form any adult cell, but are not derived from an embryo.

As used herein, "modulator" or "modulate" and all their forms and tenses (including, for example, modulation, modulating and modulated) refer to an agent that acts (or the act itself) to alter, adjust, or keep in proper measure or proportion a cellular event (including, for example, cell signalling and cell function). For example, a modulator of an activity of a polypeptide includes altering, adjusting or keeping in proper measure the activity of the polypeptide. Modulator includes both activators and inhibitors or suppressors. This definition similarly applies to "modulator" in immunomodulator.

As used herein, the term "pluripotent" refers to the potential of a stem cell to make any differentiated cell of an organism. Pluripotent stem cells can give rise to any fetal or adult cell type. However, alone they cannot develop into a fetal or adult organism because they lack the potential to contribute to extraembryonic tissue, such as the placenta.

Wnt modulator refers to a modulator of the Wnt signalling pathway which is an important pathway regulating a diverse range of biological functions in an organism.

### Detailed description of the invention

Described is a method for inducing cardiomyocyte differentiation comprising the steps of:
(i) providing at least one pluripotent stem cell;
(ii) contacting the pluripotent stem cell(s) with:
   (a) at least one p38 mitogen-activated protein kinase (p38-MAPK) modulator;
   (b) at least one immunomodulator; and
   (c) at least one Wnt modulator.

According to a related aspect, there is described a method for inducing cardiomyocyte differentiation comprising the steps of:
(i) providing at least one pluripotent stem cell;
(ii) contacting the pluripotent stem cell(s) with:
   (a) at least one p38 mitogen-activated protein kinase (p38-MAPK) inhibitor;
   (b) at least one immunosuppressant; and
   (c) at least one Wnt modulator.

Accordingly, the invention provides an in vitro method for inducing cardiomyocyte differentiation comprising the steps of:
(i) providing at least one induced pluripotent stem cell or embryoid body comprising induced pluripotent stem cells;
(ii) contacting said pluripotent stem cell(s) or said embryoid body with:
   (a) SB 203580 (4-[4-(4-fluorophenyl)-2-(4-methylsulfinylphenyl)-1H-imidazol-5-yl]pyridine;
   (b) cyclosporin-A (CSA); and
   (c) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) at a final concentration of 5 µM.

Optional features are specified by the dependent claims.

The method may comprise additionally contacting the pluripotent stem cell(s) with at least one Reactive Oxygen Species (ROS) modulator. The ROS modulator may comprise a ROS activator or a ROS inhibitor. In particular, the ROS modulator comprises a ROS activator.

The pluripotent stem cell(s) may be contacted with the p38-MAPK modulator, immunomodulator; Wnt modulator sequentially or concurrently. As an example, the pluripotent stem cell(s) may be contacted with the p38-MAPK modulator, immunomodulator; Wnt modulator sequentially, in any order. With the p38-MAPK modulator, immunomodulator and Wnt modulator, there would be 3x2x1 = 6 combinations for the order of contacting. In particular, the pluripotent stem cell(s) may be contacted with the p38-MAPK modulator; immunomodulater; and Wnt modulator, sequentially, in the order listed.

The pluripotent stem cell(s) may also be contacted with the p38-MAPK modulator, immunomodulator; Wnt modulator and ROS modulator; sequentially or concurrently. With the four components, there would be 4x3x2x1 = 24 combinations for the order of contacting. In particular, the pluripotent stem cell(s) may be contacted with the p38-MAPK modulator; immunomodulator, Wnt modulator; and ROS modulator, sequentially, in the order listed.

Alternatively, the three (p38-MAPK modulator; immunomodulator; and Wnt modulator); or four components (additionally including ROS modulator) may be contacted with the pluripotent stem cell(s) concurrently. For example, the three or four components may be separate components but contacted with the pluripotent stem cell(s) at the same time. In another example, these components may be mixed in a composition and contacted with the pluripotent stem cell(s) at the same time. The three or four components may be mixed just before contacting the pluripotent stem cells.

A related method for inducing cardiomyocyte differentiation could be used comprising contacting the pluripotent stem cell(s) with a composition comprising at least one p38 mitogen-activated protein kinase (p38-MAPK) modulator, at least one immunomodulator and at least one Wnt modulator. For example, the method comprises contacting the pluripotent stem cell(s) with a composition comprising at least one p38-MAPK inhibitor, at least one immunosuppressor; and at least one Wnt modulator.

According to another example including at least one ROS modulator, the method comprises contacting the pluripotent stem cell(s) with a composition comprising at least one p38 mitogen-activated protein kinase (p38-MAPK) modulator, at least one immunomodulator; at least one Wnt modulator and at least one ROS modulator. In particular, the method comprises contacting the pluripotent stem cell(s) with a composition comprising at least one p38-MAPK inhibitor, at least one immunosuppressor; at least one Wnt modulator and at least one ROS activator.

The method according to the invention is performed *in vitro.* Accordingly, the method is performed with isolated pluripotent stem cell(s).

The pluripotent stem cell(s) may be from any animal. For example, the pluripotent stem cells may be human. The pluripotent stem cell(s) may comprise induced pluripotent stem cell(s) or embryonic stem cell(s). In particular, the pluripotent stem cells(s) comprise induced pluripotent stem cell(s). Any method of preparing induced pluripotent stem cells is applicable for the invention. Further, the pluripotent stem cell(s) may be part of an embryoid body.

For example, the method may comprise the steps of:
(i) inducing pluripotent stem cells to form at least one embryoid body;
(ii) contacting the embryoid body with:
   (a) at least one p38 mitogen-activated protein kinase (p38-MAPK) modulator;
   (b) at least one immunomodulator; and
   (c) at least one Wnt modulator.

In particular, the method may comprise the steps of:
(i) inducing pluripotent stem cells to form at least one embryoid body;
(ii) contacting the embryoid body with:
   (a) at least one p38 mitogen-activated protein kinase (p38-MAPK) inhibitor;
   (b) at least one immunosuppressant; and
   (c) at least one Wnt modulator.

The pluripotent stem cell(s) or embryoid body may be contacted with the p38-MAPK modulator, immunomodulator; Wnt modulator sequentially or concurrently.

The pluripotent stem cell(s) may be contacted with the p38-MAPK modulator, immunomodulator; Wnt modulator and ROS activator sequentially or concurrently.

The pluripotent stem cell(s) or embryoid body may be contacted with a composition comprising the component(s). Alternatively, the component(s) as applicable may be added to a composition contacting the pluripotent stem cell(s) or embryoid body. This means of contacting is also applicable for the immunosuppressant, the Wnt modulator or the ROS activator.

As another related example, the method comprises (i) contacting the pluripotent stem cell(s) or embryoid body with a first composition comprising a p38-MAPK inhibitor, (ii) contacting the pluripotent stem cell(s) or embryoid body with a second composition comprising a p38-MAPK inhibitor and an immunosuppressant and (iii) contacting the pluripotent stem cell(s) or embryoid body with a third composition comprising a Wnt inhibitor.

As yet another example, the method comprises (i) contacting the pluripotent stem cell(s) or embryoid body with a composition B comprising a p38-MAPK inhibitor, (ii) contacting the pluripotent stem cell(s) or embryoid body with a composition C comprising a p38-MAPK inhibitor, an immunosuppressant and a Wnt inhibitor and (iii) contacting the pluripotent stem cell(s) or embryoid body with a composition D comprising a p38-MAPK inhibitor, an immunosuppressant and a Wnt inhibitor.

Composition B may additionally comprise ascorbic acid, bone morphogenic protein 4 (BMP4), activin A, and fibroblast growth factor 2 (FGF2). Composition C may additionally comprise ascorbic acid, Noggin, a transforming growth factor β (TGF-β) type I receptor activin receptor-like kinase 5 (ALK5) kinase inhibitor and vascular endothelial growth factor (VEGF). For example, the ALK5 kinase inhibitor may be A83-01.

In yet another example, Composition C may also comprise an epidermal growth factor receptor kinase inhibitor in addition to ascorbic acid, Noggin, a transforming growth factor β (TGF-β) type I receptor activin receptor-like kinase 5 (ALK5) kinase inhibitor and vascular endothelial growth factor (VEGF). For example, the epidermal growth factor receptor kinase inhibitor may be AG1478. The concentration of AG1478 may be 5 uM, for example.

Composition D may additionally comprise ascorbic acid and vascular endothelial growth factor (VEGF).

In yet another example, Composition D may also comprise an epidermal growth factor receptor kinase inhibitor in addition to ascorbic acid and vascular endothelial growth factor (VEGF). For example, the epidermal growth factor receptor kinase inhibitor may be AG1478. The concentration of AG1478 may be 5 uM, for example.

The pluripotent stem cell(s) or embryoid body may be contacted with the compositions for any suitable period from inducing the pluripotent stem cells to form embryoid bodies. For example, composition B from day 1 to day 4 post-EB induction; composition C from day 4 to day 6 post EB-induction and composition D from day 6 to day 8 post-EB induction.

Contacting the pluripotent stem cell(s) or embryoid body with a subsequent composition (e,g, a composition comprising a p38 MAPK inhibitor and an immunosuppressant) includes replacing the existing composition (e,g, a composition comprising p38-MAPK inhibitor) with the subsequent composition (e,g, a composition comprising a p38 MAPK inhibitor and an immunosuppressant).

Subsequent components may be added to the existing composition. For example, contacting the pluripotent stem cell(s) or embryoid body with a subsequent composition comprising a first component (e,g, a 38-MAP inhibitor) and a second component (e..g an immunosuppressant) may include adding the second component (e,g an immunosuppressant) to the existing composition already comprising the first component (e.g. a p38 MAPK inhibitor) or adding (more of the) first component (e.g a p38 MAP K inhibitor) and the second component (e.g. an immunosuppressant) to the existing composition (already comprising the first component). Applicable adjustments to the components are made as necessary depending on the existing composition and the subsequent composition.

Any suitable method for inducing embryoid body formation is applicable for the invention.

The method may additionally comprise contacting the pluripotent stem cell(s) or embryoid body with at least one Reactive Oxygen Species (ROS) modulator.

The pluripotent stem cell(s) or embryoid body may be contacted with the components for any suitable period from inducing the pluripotent stem cells to form embryoid bodies. For example, the p38-MAPK modulator may be contacted from day 2 through to day 8 following EB induction; the imunomodulator may be contacted from day 4 through to day 8 following EB induction; the Wnt modulator may be contacted from day 4 through to day 8 following EB induction while the ROS modulator may be contacted on day 8 following EB induction. The embryoid body may also be contacted with the three components (p38-MAPK modulator, the immunomodulator, and the Wnt modulator) or four components (additionally including the ROS modulator) concurrently on any single day from; any number of days selected from; or all of the days from; day 2 through to day 8 following EB induction.

A combination and/or kit may be provided comprising at least one p38 mitogen-activated protein kinase (p38-MAPK) modulator; at least one immunomodulator; and at least one Wnt modulator. A combination and/or kit may be provided comprising at least two components from the group consisting of the following components: a p38 mitogen-activated protein kinase (p38-MAPK) modulator; an immunomodulator; and a Wnt modulator. The combination and/or kit may additionally comprise at least one ROS modulator.

Accordingly, a combination and/or kit may be provided comprising at least one p38 mitogen-activated protein kinase (p38-MAPK) modulator; at least one immunomodulator; at least one Wnt modulator; and at least one ROS modulator. In particular, the combination and/or kit may be for use in inducing cardiomyocyte differentiation.

For example, a combination and/or kit may be provided comprising at least one p38 mitogen-activated protein kinase (p38-MAPK) inhibitor; at least one immunosuppressant; and at least one Wnt modulator. The combination and/or kit may additionally comprise a ROS modulator. For example, the combination and/or kit may additionally comprise at least one ROS activator. In particular, a combination and/or kit may be provided comprising at least one p38 mitogen-activated protein kinase (p38-MAPK) inhibitor; at least one immunosuppressant; at least one Wnt modulator; and at least one ROS activator.

As other examples, the combination and/or kit comprises at least one p38 mitogen-activated protein kinase (p38-MAPK) inhibitor and at least one immunosuppressant; at least one mitogen-activated protein kinase (p38-MAPK) inhibitor and at least one Wnt modulator or at least one immunosuppressant; and at least one Wnt modulator. The combination and/or kit may additionally comprise at least one ROS modulator.

There may be provided a composition comprising at least one p38-MAPK modulator; at least one modulator; and at least one Wnt modulator. There may be provided a composition comprising at least two components from the group consisting of the following components: a p38 mitogen-activated protein kinase (p38-MAPK) modulator; an immunomodulator; and a Wnt modulator. The composition may further comprise at least one ROS modulator. In particular, there may be provided a composition comprising at least one p38 mitogen-activated protein kinase (p38-MAPK) inhibitor; at least one immunosuppressant; at least one Wnt modulator; for use in inducing cardiomyocyte differentiation. The composition may further comprise a ROS modulator, for example a ROS activator. The composition may be for use in inducing cardiomyocyte differentiation.

The invention may also include Composition B, C, and/or D as defined in the claims. As other examples, the combination and/or kit comprises at least one p38 mitogen-activated protein kinase (p38-MAPK) inhibitor and at least one immunosuppressant; at least one mitogen-activated protein kinase (p38-MAPK) inhibitor and at least one Wnt modulator or at least one immunosuppressant; and at least one Wnt modulator. The combination and/or kit may additionally comprise at least one ROS modulator.

At least one p38 mitogen-activated protein kinase (p38-MAPK) modulator; at least one immunomodulator; and at least one modulator may be used for the preparation of a combination or composition for inducing cardiomyocyte differentiation. For example, at least one p38 mitogen-activated protein kinase (p38-MAPK) inhibitor; at least one immunosuppressant; and at least one Wnt modulator may be used for the preparation of a combination or composition for inducing cardiomyocyte differentiation.

At least two components selected from the group consisting of the following components: a p38-MAPK inhibitor, an immunosuppressant and a Wnt modulartor may be used for the preparation of a combination or composition for inducing cardiomyocyte differentiation.

Aat least one p38 mitogen-activated protein kinase (p38-MAPK) modulator; at least one immunomodulator; at least one Wnt modulator; and at least one ROS modulator may be used for the preparation of a combination or composition for inducing cardiomyocyte differentiation.

In particular, at least one p38 mitogen-activated protein kinase (p38-MAPK) inhibitor; at least one immunosuppressant; at least one Wnt modulator and at least one ROS activator may be used for the preparation of a combination or composition for inducing cardiomyocyte differentiation.

The combination, kit or composition may be for use in inducing cardiomyocyte differentiaton from at least one pluripotent stem cell.

Any suitable amount or concentration of each of the components (p38-MAPK modulator; immunomodulator; Wnt modulator and/or ROS modulator) may be used.

The p38-MAPK modulator may be an activator or an inhibitor. In particular, the p38-MAPK modulator comprises a p38-MAPK inhibitor. For example, the p38-MAPK modulator includes but is not limited to SB 203580 (4-[4-(4-fluorophenyl)-2-(4-methylsulfinylphenyl)-1H-imidazol-5-yl]pyridine), BIRB 796 1-[5-tert-butyl-2-(4-methylphenyl)pyrazol-3-yl]-3-[4-(2-morpholin-4-ylethoxy)naphthalen-1-yl]urea), SB 202190 (4-[4-(4-fluorophenyl)-5-pyridin-4-yl-1,3-dihydroimidazol-2-ylidene]cyclohexa-2,5-dien-1-one, VX-702 6-(N-carbamoyl-2,6-difluoroanilino)-2-(2,4-difluorophenyl)pyridine-3-carboxamide), LY2228820 (5-[2-tert-butyl-4-(4-fluorophenyl)-1H-imidazol-5-yl]-3-(2,2-dimethylpropyl)imidazo[4,5-b]pyridin-2-amine; methanesulfonic acid) and VX-745 (5-(2,6-dichlorophenyl)-2-(2,4-difluorophenylthio)-6H-pyrimido[1,6-b]pyridazin-6-one) and PH-797804 (3-[3-bromo-4-[(2,4-difluorophenyl)methoxy]-6-methyl-2-oxopyridin-1-yl]-N,4-dimethylbenzamide).

Similarly:
The immunomodulator may comprise an immunoactivator or an immunosuppressant. In particular, the immunomodulator comprises an immunosuppressant. For example, the immunomodulator includes but is not limited to cyclosporin-A (CsA), tacrolimus and sirolimus.

The Wnt modulator modulates the Wnt pathway, either activating or inhibiting the Wnt pathway. Accordingly, the Wnt modulator may comprise an activator or an inhibitor. For example, the Wnt modulator includes but is not limited to IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide), IWP3 (2-(3-(4-fluorophenyl)-3,4,6,7-tetrahydro-4-oxothieno[3,2-d]pyrimidin-2-ylthio)-N-(6-methylbenzo[d]thiazol-2-yl)acetamide), IWP2 (N-(6-Methyl-2-benzothiazolyl)-2-[(3,4,6,7-tetrahydro-4-oxo-3-phenylthieno[3,2-d]pyrimidin-2-yl)thio]-acetamide), pyrvinium (2-[(E)-2-(2,5-dimethyl-1-phenylpyrrol-3-yl)ethenyl]-N,N,1-trimethylquinolin-1-ium-6-amine, BIO ((3Z)-6-bromo-3-[3-(hydroxyamino)indol-2-ylidene]-1H-indol-2-one), CHIR99021 (6-[2-[[4-(2,4-dichlorophenyl)-5-(5-methyl-1H-imidazol-2-yl)pyrimidin-2-yl]amino]ethylamino]pyridine-3-carbonitrile) and XAV939 (2-[4-(trifluoromethyl)phenyl]-1,5,7,8-tetrahydrothiopyrano[4,3-d]pyrimidin-4-one).The method comprises contacting the pluripotent stem cell(s) or embryoid body with the p38-MAPK inhibitor 4-[4-(4-fluorophenyl)-2-(4-methylsulfinylphenyl)-1H-imidazol-5-yl]pyridine (SB 203580); the immunosuppressant cyclosporin-A (CSA); and the Wnt modulator IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide).

Any suitable ROS modulator may be used. The ROS modulator may comprise a ROS activator or ROS inhibitor. In particular, the ROS modulator comprises a ROS activator. For example, the ROS activator comprises hydrogen peroxide (H₂O₂).

Having now generally described the invention, the same will be more readily understood through reference to the following examples which are provided by way of illustration, and are not intended to be limiting of the present invention.

### EXAMPLES

Standard molecular biology techniques known in the art and not specifically described were generally followed as described in Molecular Cloning: A Laboratory Manual, Cold Springs Harbor Laboratory, New York.

### Reference example 1

Four agents (a p38-MAPK inhibitor; an immunosuppressant; a Wnt modulator; and a ROS activator) were evaluated for their effects on cardiac differentiation.

### Materials and methods

### Human embryonic stem (ES) cells and induced pluripotent stem cell (iPSC) cultures

Human ES cells (H9; Wicell, USA) and iPS cells (MSnviPSNF2 (C2) and MSnviPSNF3 (C3); both described in Mehta *et al.,* 2011) were maintained on 1 % matrigel (BD Biosciences, CA, USA) and grown in chemically defined mTeSR1 medium (Stem Cell Technologies, VA, Canada). Differentiated areas (<10%) were manually removed periodically with a scraper and prior to passaging. Pluripotent stem cells were subsequently washed with DMEM-F12 (Invitrogen, CA, USA) and incubated with 1 mg/ml dispase (Stem Cell Technologies, VA, Canada) for 5-7 minutes. Subsequently, cells colonies were washed using DMEM-F12 and carefully cut into small clumps of approximately 50-70 µm in size before being scrapped from the surface of the culture dishes. Colonies were later re-plated onto new matrigel coated dishes. mTeSR medium is changed daily for the newly plated cells and passaged every 7 days. Pluripotent stem cells were maintained in a 37°C environment under 5% CO₂ in air at 95% humidity.

### Cardiac differentiation

Embryoid body (EB) generation is a robust method used in human embryonic stem cells (hESCs) and human induced pluripotent stem cells (hiPSCs) differentiation. Generally, dissociated hiPSC/hESC aggregate together in a suspension system to form EBs. These EBs mimic post-implantation embryos and can be induced to differentiate into cells of the three embryonic germ layers.

On day 0, pluripotent stem cell colonies (H9, C2 and C3) were dispersed into small clumps using dispase (1 mg/mL) and placed in low-adhesion culture dishes in embryoid body (EB) medium supplemented with 20% Fetal Bovine Serum as described previously (Mehta *et al.,* 2011) to prepare embryoid bodies. Other methods to prepare embryoid bodies, may also be used

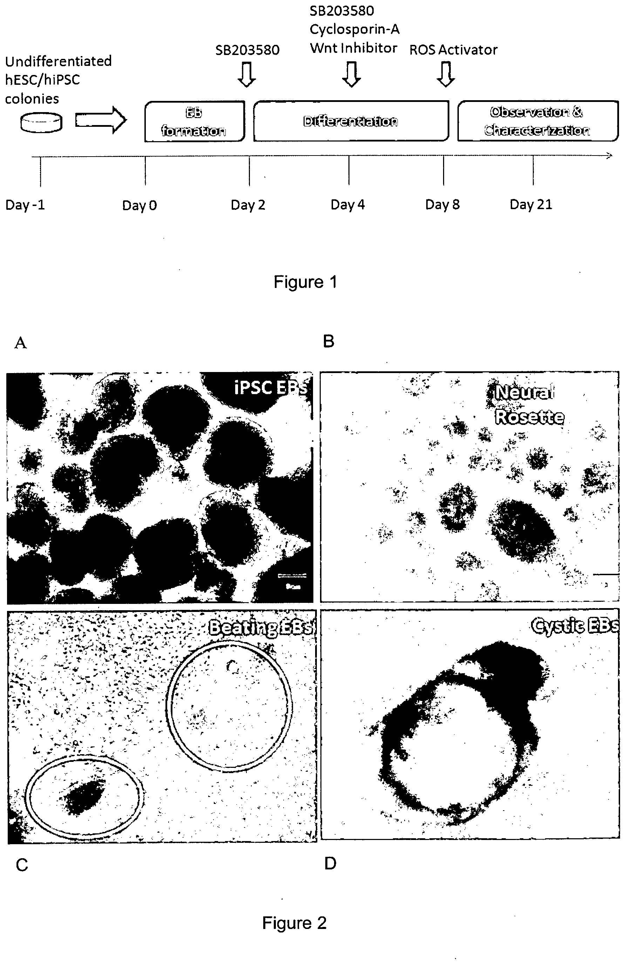

Subsequently, addition of different small molecules (components) were carried out at various stages of differentiation, either alone or in combination with at least one other component (Figure 1): SB 203580 daily from day 2 through to day 8 from EB induction; Wnt modulator IWP4 daily from day 4 through to day 8 from EB induction, CSA daily, from day 4 through to day 8 from EB induction, and ROS activator H₂O₂ on day 8 (15 min prior to plating). EBs were then plated on 0.1% gelatin-coated dishes in EB medium without the molecules. Plated EBs were periodically screened for contracting loci. All EBs were evaluated morphologically under the microscope at 4x magnification for 10-15 seconds. EB with minimally one beating foci is considered as a beating cluster. Beating areas were typically observed around days 10 to 16 after EB formation. Final evaluations were performed on day 21.

### Reverse Transcription PCR (RT-PCR)

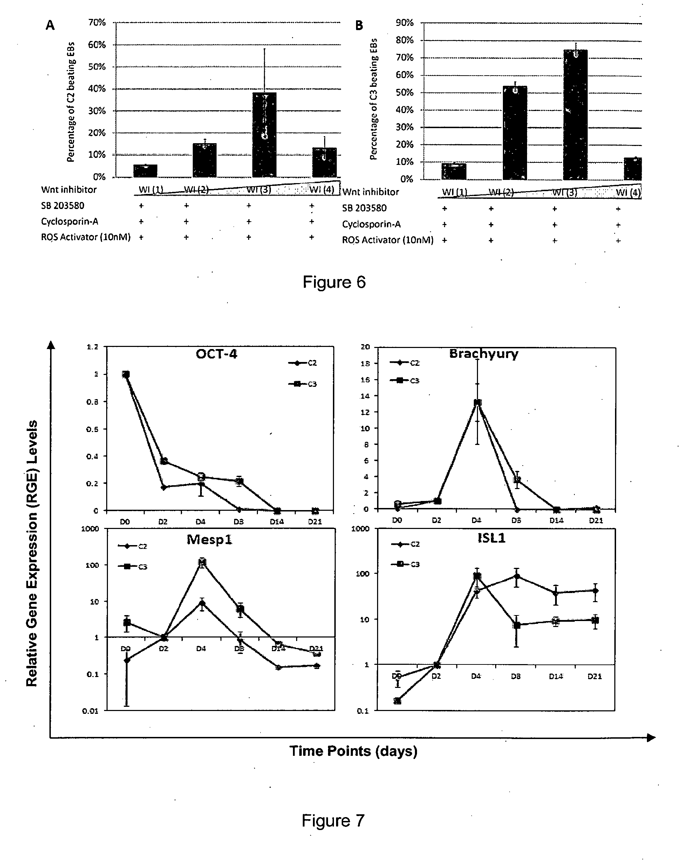

Total RNA was extracted from samples using RNeasy Mini Kit (QIAGEN) according to the manufacturer's instruction. A total of one microgram of cDNA was synthesized from isolated RNA and qPCR performed as described previously (Mehta *et al.,* 2011). Gene expression of selected markers in the C2 and C3 cell lines was studied. Quantitative real-time PCR data of the selected markers was performed at six time points (Day 0, 2, 4, 8, 14, 21). The mean Ct values of duplicate measurements were determined and normalized against housekeeping gene (GAPDH) for each sample in the same run. All relative gene expression levels were calculated using the "ΔΔCt formula" for differentiated markers with respect to day 2 samples, except for Oct4 which was calculated using day 0 samples. For both cell lines, the means of duplicate samples from two independent experiments depicted in Figures 7-10 are means ± SEM (n=2). The primers used for the quantitative real-time PCR are shown in Table 1.

**Table 1 Primers for quantitative real-time PCR**

| **Gene** | **Forward** | **Reverse** |
|---|---|---|
| GAPDH | 5' GTGGACCTGACCTGCCGTCT 3' (SEQ ID NO: 1) | 5' GGAGGAGTGGGTGTCGCTGT 3' (SEQ ID NO: 2) |
| OCT-4 | 5' AGTTTGTGCCAGGG TTTI I G 3' (SEQ ID NO: 3) | 5' ACTTCACCTTCCCTCCAACC 3' (SEQ ID NO: 4) |
| GATA-4 | 5' TCCAAACCAGAAAACGGAAG 3' (SEQ ID NO: 5) | 5' AAGGCTCTCACTGCCTGAAG 3' (SEQ ID NO: 6) |
| ISL-1 | 5' AAGGACAAGAAGAGAAGCAT 3' (SEQ ID NO: 7) | 5' CATGGGAGTTCCTGTCATCC 3' (SEQ ID NO: 8) |
| Brachyury (T) | 5' TCGGGGCCCACTGGATGA 3' (SEQ ID NO: 9) | 5' ATGCGCTGTGGACCCCCA 3' (SEQ ID NO: 10) |
| HAND1 | 5' AAGCTTTCCCTGTGTTGGAA 3' (SEQ ID NO: 11) | 5' GGCAGGATGAACAAACACCT 3' (SEQ ID NO: 12) |
| MESP1 | 5' GGCAGAGGCAGAGCGCCA 3' (SEQ ID NO: 13) | 5' AGCGGATAGCCAGGCGCAG 3' (SEQ ID NO: 14) |
| MEF-2C | 5' CCCAACCTATTGCCACTGGCT 3' (SEQ ID NO: 15) | 5' ATACCCGTTCCCTGCACTGGT 3' (SEQ ID NO: 16) |
| NKX 2.5 | 5' CTAAACCTGGAACAGCAGCA 3' (SEQ ID NO: 17) | 5' GTAGGCCTCTGGCTTGAAGG 3' (SEQ ID NO: 18) |
| TBX5 | 5' TCTGTGACGGGCAAAGCTGAG 3' (SEQ ID NO: 19) | 5' AATATGCCCAAATGGGTCCAGGT (SEQ ID NO: 20) |
| TBX20 | 5' AGTGGCAGCAGCCCGTCCT 3' (SEQ ID NO: 21) | 5' CATCTCGGTGCCCAGCTCATG 3' (SEQ ID NO: 22) |
| CACNA1D | 5' GGGCAATGGGACCTCATAAATAA 3' (SEQ ID NO: 23) | 5'TTACCTGGTTGCGAGTGCATTA 3 (SEQ ID NO: 24) |
| RYR2 | 5' CAGGAAGTGAGGCAGCCCAA 3' (SEQ ID NO: 25) | 5'CAGACACAGCGCCACCTTCATA 3 (SEQ ID NO: 26) |
| HCN2 | 5' CACCTGCTACGCCATGTTCA 3' (SEQ ID NO: 27) | 5' CTGGCAGCTTGTGGAAGGA 3' (SEQ ID NO: 28) |
| KCNH2 | 5' CTGATCGGGCTGCTGAAGAC 3' (SEQ ID NO: 29) | 5' AGCCAATGAGCATGACGCA 3' (SEQ ID NO: 30) |
| SERCA2 | 5'CCAAAGTCATCTCCCTTATTTGCATT (SEQ ID NO: 31) | 5' GACCTTCAGGAATGGCTGCTACA 3' (SEQ ID NO: 32) |
| MLC2V | 5' CCTTGGGCGAGTGAACGT 3' (SEQ ID NO: 33) | 5' GGGTCCGCTCCCTTAAGTTT 3' (SEQ ID NO: 34) |
| MHY7 | 5' GGCAAGACAGTGACCGTGAAG 3' (SEQ ID NO: 35) | 5'CGTAGCGATCCTTGAGGTTGTA 3 (SEQ ID NO: 36) |
| CTnl | 5' CCAACTACCGCGCTTATGC 3' (SEQ ID NO: 37) | 5' CTCGCTCCAGCTCTTGCTTT 3' (SEQ ID NO: 38) |

### Results

### Treatment with p38-MAPK inhibitor alone is insufficient in directing cardiogenesis in hiPSC cell-lines (C2 and C3).

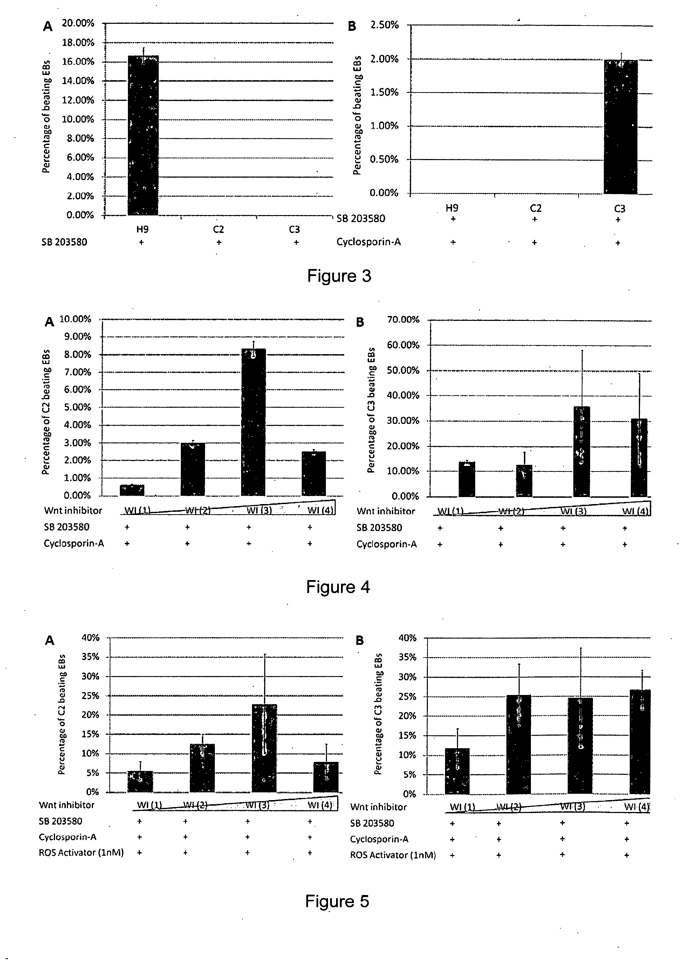

To understand and determine the differentiation efficiency of SB203580, one hESC (H9) cell line; and two hiPSC (C2 and C3) cell lines reprogrammed from human foreskin fibroblasts were selected. Utilising the EB suspension system, all cell lines were able to form EBs after 1 day in EB20 medium (Figure 2A). Following addition of SB203580 to a final concentration of 5 µM in the culture medium from day 2 through to day 8, spontaneous beating clusters (Figure 2C and figure 3A) were observed in the hESC (H9) cell line as early as day 14 and 16.6% of total EBs plated were found to be beating after 21 days. However, derivatives of the ectoderm (neuroepithelial rosettes) (Figure 2B), mesoderm (mesenchymal stem cell-like cells) and endoderm-like cells were identified for iPSC (C2 and C3) cell lines after addition of SB203580 under the microscope. Cystic EBs (Figure 2D) were also observed with the SB203580 treated iPSC (C2 and C3) cell lines. Consequently, no cardiac-inducing effect was observed for both iPSC clones (C2 and C3) even after 21 days post-differentiation with SB203580 treatment (Figure 3A).

### Treatment with p38-MAPK inhibitor and Immunosuppressant, Cyclosporin-A (CSA) moderately induce cardiomyogenesis in hiPSC cell line (C3).

The effect of SB203580 and cyclosporin-A treatment was investigated. Treatment with 5 µM (final concentration in culture medium) of SB203580 daily from day 2 through to day 8; and 3 µg/ml of CSA (final concentration in culture medium daily from day 4 through to day 8 was insufficient to induce cardiac cell formation in the hESC (H9) and hiPSC (C2) cell lines (Figure 3B). However, a few spontaneously beating EBs were observed when the hiPSC C3 cell line was similarly treated (Figure 3B). It appears that an additive effective of CSA in cardiomyogenesis could have been present but this was inadequate to efficiently induce diiferentiation of cardiomyocytes in the hESC (H9) and hiPSC (C2) cell lines.

### Treatment with p38-MAPK inhibition, CSA treatment and Wnt/b-catenin pathway modulation significantly increase cardiomyogenesis

The effect of including a Wnt modulator IWP4 was investigated. EB generation was repeated for the hESC (H9) and hiPSC (C2 and C3) cell lines as described above. SB203580 was added to a final concentration of 5 µM in the culture medium daily from day 2 through to day 8, CSA was added to a final concentration of 3 µg/ml in the culture medium daily from day 4 through to day 8. A titration study of four concentrations (final concentrations of WI(1):1 µM; WI(2): 3 µM; WI(3): 5 µM and WI(4): 7 µM of Wnt modulator IWP4 in the culture medium; added daily from day 4 through to day 8, was performed. After the addition of SB203580, CSA and Wnt modulator IWP4, EBs were subsequently plated on day 8 for observation of contracting loci as described above. Beating clusters were observed as early as 14 days and 11 days post-differentiation for the C2 and C3 cell lines respectively.

On day 21, the percentage of beating EBs for the C2 cell line was between 0.63 % and 8.33 % (Figure 4A) whereas the percentage for the C3 cell line was between 12.71 % and 35.87 % (Figure 4B); depending on the concentration of Wnt modulator used. This study revealed that the enhancing ability of Wnt modulator IWP4 in inducing contracting loci peaked at WI(3): 5 µM for both the hiPSC cell lines, C2 and C3. In contrast, EBs that were subjected to lower concentrations of the Wnt modulator IWP4, WI(1): 1 µM and WI(2): 3 µM, appeared to have differentiated into other cell lineages. Notably, the majority of EBs from Wnt modulator IWP4 concentrations of WI(1): 1 µM and WI(2): 3 µM formed neural rosettes, neurons, endothelial cells, tubular aggregates and fibroblasts. This observation was higher in EBs from the C2 cell line.

### Including ROS activator induces a higher number of cardiac beating clusters in both hiPSC cell lines (C2 and C3).

The treatment with SB203580 (final concentration of 5 µM in culture medium daily from day 2 through to day 8), CSA (final concentration of 3 µg/ml in culture medium daily from day 4 through to day 8) and different concentrations of Wnt modulator IWP4 (daily from day 4 through to day 8) was as described above. Subsequently, ROS activator, H₂O₂ was added on day 8 at two different final concentrations (1 nM and 10 nM) in the culture medium for 15 minutes prior to plating. Both concentrations of ROS activator could maintain or increase the number of contracting clusters across all four concentrations of Wnt modulator IWP4 with SB203580 and CSA.

Specifically, ROS activator at a final concentration of 1 nM in the culture medium was able to induce an increase in the average beating clusters of the C3 cell line by between 5.67% and 22.86% (Figure 5A). 1 nM of ROS activator maintained the average beating cluster at between 11.76% and 26.67% with the different concentrations of Wnt modulator IWP4 (Figure 5B).

With the final concentration of 10 nM of ROS activator in the culture medium, there was no significant effect on beating clusters in the C2 cell line also treated with first two components (SB203580 and CSA) and the Wnt modulator IWP4 at a concentration of WI(1): 1 µM in the culture medium (Figure 6A), However, an increase in the number of beating clusters was observed in the C2 cell line treated with the first two components and the other three concentrations of Wnt modulator IWP4.

ROS activator at a final concentration of 10 nM in the culture medium was also observed to enhance the percentage of beating clusters in the C3 cell lines treated with the first two components and the Wnt modulator IWP4 at final concentrations of WI(2): 3 µM and WI(3): 5 µM in the respective culture medium (Figure 7B). A drop in the percentage of beating clusters was observed with the C3 cell line treated with the first two components, Wnt modulator IWP4 at a final concentration of WI(4): 7 µM and ROS activator at a final concentration of 10 nM; in the culture medium (Figure 7B).

Based on this study, the combination comprising SB203580 at a final concentration of 5 µM; Cyclosporin-A at a concentration of 3 µg/ml Wnt modulator IWP4 at a final concentration of WI(3): 5 µM and ROS activator at a final concentration of 10 nM; in the culture medium was the most efficient in inducing cardiomyogenesis in both the C2 and C3 cell lines, with 38.33% and 75% of beating clusters for the C2 and C3 cell lines, respectively.

### Temporal Gene Expression Patterns during cardiomyogenesis

Temporal gene expression was investigated using time point collection and quantitative PCR (qPCR). The time points were selected with an understanding to help in evaluating key time frames during cardiomyogenesis, so that early, intermediate as well as late stages of cardiomyogenesis could be studied.

The present differentiation protocol was used to characterize development ontogeny of hiPSC-derived cardiomyocytes through hiPSC cardiac gene expression patterns and this could provide information for optimising the efficiency of hiPSCs cardiac differentiation for clinical and pharmacological applications. By knowing which genes are up or down-regulated at different time points of cardiomyogenesis could enable the targeting of more specific genes in hiPSCs to direct them towards cardiomyogenic lineage. A panel of eighteen markers were used in quantitative polymerase chain reaction (qPCR) studies to evaluate the expression of pluripotent genes, mesodermal and cardiac mesodermal genes, followed by cardiac-specific transcription factors and structural genes, involved in the process of cardiomyogenesis in hiPSC cell lines C2 and C3.

### Gene expression in hiPSC C3 cell line

Expression level of pluripotent state-specific transcription factor Oct-4 decreased immediately after growing the cells in EB suspension cultures, especially with a significant drop of 2.5 folds from day 0 to day 2 (d0 vs d2: 1.00±0.00 vs 0.37±0.01, p<0.05, n=2) as illustrated on Figure 7. In contrast, the other markers Brachyury, Mesp1 and ISL1 showed an increase in expression levels from Day 0 to Day 2, indicating that the cells were shifting from undifferentiated state towards differentiation process (Figure 7).

A significant up regulation of 10-15 folds and 100 folds was observed for Brachyury (d2 vs d4: 1.00 ±0.00 vs 13.19±2.26, p<0.05, n=2) and Mesp1 (d2 vs d4: 1.00±0.00 vs 120.95±39.94, p<0.05, n=2), respectively on day 4 of differentiation. Furthermore, the levels of both these markers dropped significantly post-day 4 of differentiation and were not expressed on other days of differentiation (Figure 7).

It was interesting to note that the decrease of Brachyury and Mesp1 was associated with significant up regulation of cardiac progenitor markers and cardiac-associated transcription factors. For example, one of the cardiac progenitor markers used here is ISL1, which is a marker of secondary heart field. A substantial 90 fold increase in levels ISL1 by post day 4 (d2 vs d4: 1.00±0.00 vs 90.47±42.97, p<0.05, n=2), which decreased slightly and maintained its expression level till day 21 of differentiation process (Figure 7).

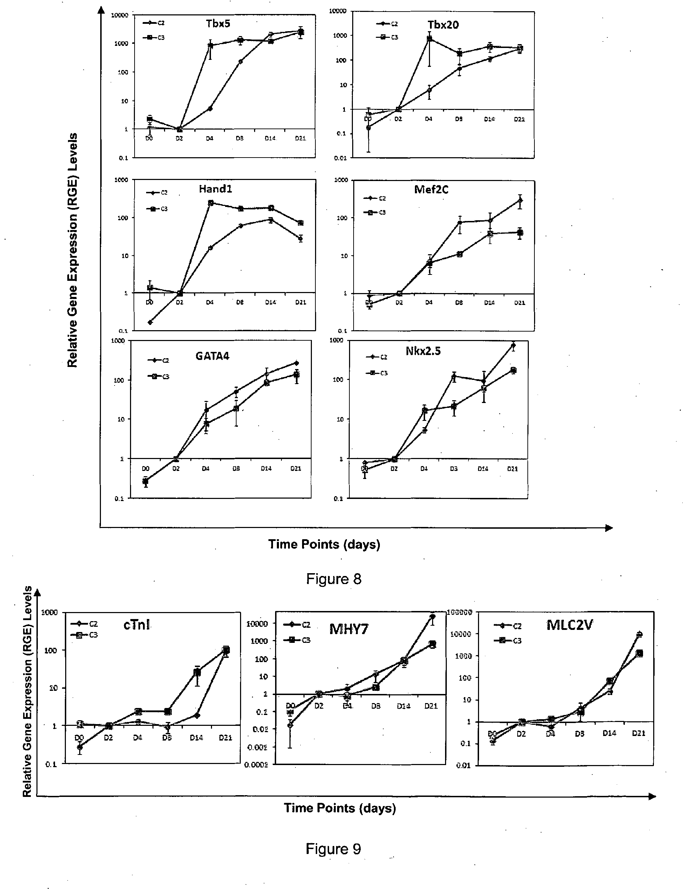

Cardiac-associated transcription factors, GATA-4, Nk2 homeobox locus 5(Nkx2.5), myocyte enhancing factor (Mef2C), heart and neural crest derivatives-expressed protein 1 (HAND1), T box factor (Tbx5, Tbx20) showed varying but substantial increase in expression post day 4 of differentiation. RGE of GATA-4 (d2 vs d4: 1.00±0.00 vs 7.64±3.29, p<0.05, n=2) and Nkx2.5 (d2 vs d4: 1.00±0.00 vs 16.57±7.18, p<0.05, n=2) both increased constantly over 7 folds and 16 folds, respectively, during the course of differentiation process (Fig. 5). Tbx5 and Tbx20 also increased 850 folds and 750 folds, respectively, by day 4 and maintained their gene expression levels throughout the differentiation process. As for Mef2C and HAND1, they both showed approximately 100 fold increase during differentiation process. HAND1 also demonstrated a downward trend in expression over the period of 21 days (Figure 8).

The expression levels of cardiac specific structural genes including cardiac troponin-I (cTnl), β-myosin heavy chain (MHY7) and myosin light chain 2V (MLC2V) were also investigated. Cardiac troponin I expression increased after day 8 (d8 vs d21: 2.43±0.33 vs 103.61±8.60, p<0.05, n=2), while MHY7 (d8 vs d21: 2.57±0.54 vs 702.66±266.11, p<0.05, n=2) and MLC2V (d8 vs d21: 2.76±1.68 vs 1285.51±84.53, p<0.05, n=2) demonstrated a gradual rise initially, which increased exponentially post day 2 onwards by more than 1000 folds (Figure 9).

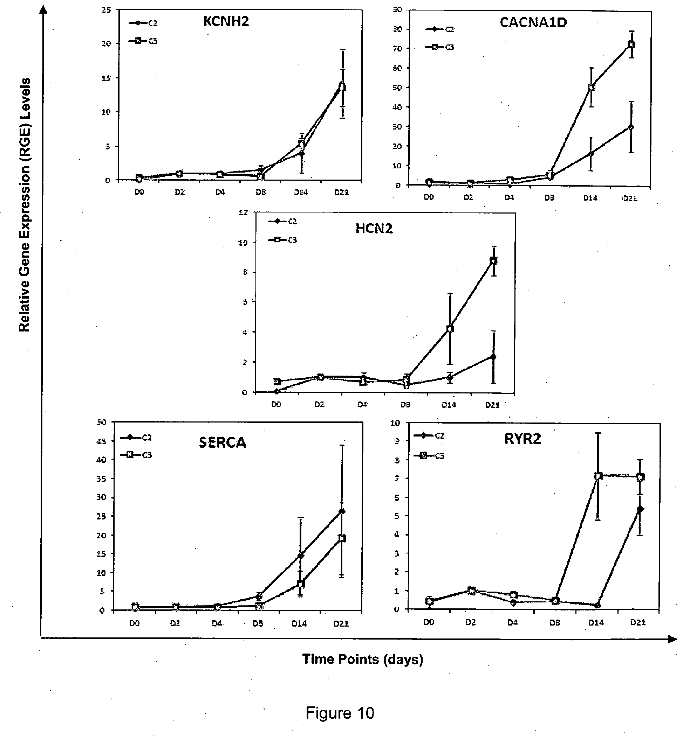

Considering that the expression of ion channels is one of the most important aspects of cardiac physiology, the expression of a panel of ion-channel encoding genes such as L-type calcium channel Caᵥ1.3 (CACNA1D), potassium-gated channel (KCNH2) and nucleotide-gated channel (HCN2), sarcoplasmic reticulum calcium ATPase (SERCA). On post day 8 of differentiation, CACNA1D exhibited 10-fold increase in expression levels, while KCNH2 and HCN2 demonstrated 15-fold and 8-fold increase in expression levels respectively. These genes demonstrated maximal expression on day 21 of differentiation. Cardiac RYR2 expression level only increased significantly on day 14 of differentiation, which makes RYR2 a relatively late marker of cardiomyogenesis (d8 vs d14: 0.49±0.14 vs 7.17±2.35, p<0.05, n=2) Lastly, SERCA expression was prominently observed only post day 8 of differentiation. The expression level of this marker too was maximally expressed in day 21 samples (Figure 10).

### Discussion

Overall, the use of p38-MAPK inhibitor (SB203580), (immunosuppressant (CSA) and Wnt modulator (IWP4) resulted in differentiation of hiPSC into cardiomyocyte. In particular, a high concentration of Wnt modulator IWP4 at WI(3): 5µM drastically increases cardiomyocyte induction.

It was also observed that the addition of p38-MAPK inhibitor (SB203580), immunosuppressant (CSA), Wnt modulator (IWP4) and ROS activator (H₂O₂) could enhance cardiomyogenesis in hiPSC.

A few significant variations were observed between the C2 and C3 cell lines. Beating clusters were detected earlier in plated EBs from the C3 cell line (day 10-12) than plated EBs from the C2 cell line (days 14-15). Thus, the expression of mesodermal markers, cardiac-specific mesodermal markers and transcription factors, as well as mature structural markers should increase faster in C3 than in C2. The expression of Oct-4 upon induction should also drop faster in C3 than in C2 (Figure 7). Interestingly, it was observed that a more pronounced down regulation of Oct-4 (C2d2 vs C3d2: 0.17±0.00 vs 0.37±0.01, p<0.05, n=2) in C2 than in C3, which may indicate that undifferentiated C2 cells have higher tendency to lose the state of pluripotency upon EB formation.

However, the expressions of other markers were consistent with early observations of beating clusters in C3. Lower fold expression was observed for intermediate markers in C2 than in C3 post day 4 of differentiation (Figure 7). From day 2 to day 4, cardiac mesodermal marker Mesp1 demonstrated a 10-fold increase in C2, while 100 folds increase was observed in C3 from day 2 (C2d2 vs C3d2: 1.00±0.00 vs 1.00±0.00, p<0.05, n=2) to day 4 (C2d4 vs C3d4: 9.12±3.62 vs 120.95±39.94, p<0.05, n=2) (Figure 7). Expression of cardiac progenitor marker ISL1 in C2 had also reached maximum expression levels only on day 8 of differentiation (C2d4 vs C3d4: 41.49±11.22 vs 90.47±42.97, p>0.5, n=2) (C2d8 vs C3d8: 92.15±39.44 vs 7.63±5.10, p<0.5, n=2), while C3 reached its maximum expression levels on day 4 of differentiation (Fig. 4). Both transcription factors Tbx5 and Tbx20 increased 10 folds in C2, while the expressions doubled by 100 folds in C3 (Figure 8). Heart and neural crest protein HAND1 (C2d2 vs C3d2: 1.00±0.00 vs 1.00±0.00, p<0.05, n=2) (C2d4 vs C3d4: 16.03±1.00 vs 250.90±37.12, p<0.05, n=2) also exhibited similar RGE levels as Mesp1, Tbx5 and Tbx20.

The same trend was observed in the expressions of cardiac troponin I (cTnl) (Figure 9) and ion channels, such as HCN2, RYR2 and CACNA1D (Figure 10). These genes demonstrated a higher increase in fold expressions in C3 than in C2. cTnl RGE levels increased 10-fold in C3. differentiating cells by day 14, but in C2 the expression levels increased more gradually. However, by day 21 both cell lines attained similar gene expression levels (C2d21 vs C3d21: 90.71±23.06 vs 103.61±8.60, p<0.05, n=2). The most significant difference between C2 and C3 is the RGE levels of CACNA1D. A 10 fold increase in CACNA1D expression levels for C3 (C3d8 vs C3d14: 5.71±2.13 vs 50.88±10.10 p<0.05, n=2), while C2 only exhibited a 4 fold increase instead (C2d8 vs C3d14: 4.27±0.38 vs 16.44±8.49, p<0.05, n=2). However, cardiac-associated markers (Mef2C, GATA-4 and Nkx2.5) (Figure 8) and cardiac specific structural genes (MHY7 and MLC2V) (Figure 9) gene expressions were surprisingly higher for C2 than in C3 differentiating cells from day 2 to day 21.

Differentiation efficiency being cell line dependent has been documented in the literature for hESC (Moore *et al.,* 2008). Similarly, the two hiPSC cell lines (C2 and C3) demonstrated disparate cardiomyocyte differentiation efficiency in the presence of SB203580. However, both responded to the induction protocol with SB203580, Cyclosporin-A, Wnt modulator IWP4 and/or ROS activator H₂O₂. Moreover, the current protocol with four agents SB203580, CSA, Wnt modulator IWP4 and ROS activator H₂O₂ has further been tested on an additional 6 different lines of hiPSC in the laboratory, with the percentage of beating EBs ranging from 20 to 45%.

### Reference example 2

The media composition and timeline of differentiation was refined to further enhances the efficiency and purity of cardiomyocytes produced and shows that a regimental transitional steering of signaling pathways in WNTs and BMPs induce efficient cardiac differentiation across various pluripotent stem cell lines that obviates tailored calibration of cytokines for individual PSCs.

### Material and Methods

### Cell lines and maintenance

A total of 9 different pluripotent stem cell lines (1 hESC and 8 hiPSC) were used in this study. Eight hiPSC lines were generated in-house or obtained from different laboratories that were derived from fibroblast from different sources of normal or diseased patients and reprogramed by various methods. Human ES line, H3-Nkx2.5-GFP, H3G, obtained from Dr David Elliot, Monash Universtity, Australia, dermal fibroblast-derived virally reprogramed hiPSC lines, 10⁺ and 8⁻, obtained from Dr Allan Colman, IMCB, Singapore. All other lines were generated in-house by episomal based systems as previously reported (Mehta *et al.,* 2011), and these include, neonatal fibroblast-hiPSC line, C3, Long QT syndrome 2-hiPSC lines, K1.3 and K1.4, atrial fibroblast-hiPSC, AT and normal patient adult fibroblast-hiPSC line, I4 and BJ fibroblast-derived-mRNA reprogramed hiPSC line, BJ.

Human ES cells and iPS cells were maintained on 1% matrigel (BD Biosciences, CA, USA) and grown in chemically defined mTeSR1 medium (Stem Cell Technologies, VA, Canada) as described previously. Pluripotent stem cells were washed with DMEM-F12 (Invitrogen, CA, USA) and incubated with 1 mg/ml dispase (Stem Cell Technologies, VA, Canada) for 5-7 minutes, washed and colonies were scrapped. Controlled tituration of the colonies was performed to get small clumps of approximately 50-70 µm in size for replating on new matrigel coated dishes (1:6 split ratio). Cells were fed daily with fresh mTeSR1 medium and passaged every 5-6 days and were maintained at 37°C under 5% CO2 in air at 95% humidity.

### Cardiac differentiation

A schematic representation of the protocol with growth factors has been shown in Figure 12A. Briefly, hiPSC/hESC lines were pre-treated with 10 µM Y27632 (Stem Cell Technologies, VA, Canada) for 4 h before dissociating them to single cells with Accutase (Stem Cell Technologies, VA, Canada). Cells were aggregated in spin EBs of 5000 cells/EB in AggreWellTM 800 (Stem Cell Technologies, VA, Canada) in media A (mTesR1: DMEM-F12-B27 (1:1) with 4mg/mL PVA) with Ascorbic acid (284 µM) and BMP4 (770 pM) and centrifuged at 300g for 5 min and incubated overnight. Next day (day 1), EBs were harvested and collected in low attachment dishes (Corning, USA) in medium B (mTesR1: DMEM-F12-B27 (1:4) with 4mg/mL PVA) with Ascorbic acid (284 µM), BMP4 (1.5 nM), Activin A (1.5 nM), SB203580 (5 µM) and FGF2 (3.1 nM) till day 4. Media was replaced with medium C (DMEM-F12-B27 with 4mg/mL PVA) along Ascorbic acid (284 µM), SB203580 (5 µM), Noggin (4.3 nM), A83-01 (1 µM), VEGF (1.5 nM), Cyclosporin A (2.5 µM) and IWP4 (10 µM) till day 6. For the last two days, EBs were cultured in medium D with Ascorbic acid (284 µM), SB203580 (5 µM), VEGF (521 pM), Cyclosporin A (2.5 µM) and IWP4 (10 µM). EBs were plated on day 8 in EB2 medium (DMEM-F12 with 2% FBS) on 0.1% gelatin coated dishes. Beating areas were clearly observed between day 10-14 depending on different cell lines. Beating areas were manually dissected out after day 14 of differentiation and utilized for all experiments.

### Immunofluorescence

Colonies of iPSC and single cells generated from beating clusters were seeded on matrigel-and gelatin-coated glass slides respectively, while whole EB staining were performed in suspension. All cell types were fixed with 4% paraformaldehyde, permeabilized with 1% Triton X-100 (Sigma-Alrich, MO, USA) and blocked with 5% bovine serum albumin (Sigma-Alrich, MO, USA). Human iPS colonies were stained for 1 hour with primary antibodies targeting pluripotency markers, Oct-4, SSEA4, Tra-1-60 and Tra-1-80 (all Millipore, MA, USA), whereas cardiomyocytes (CMs) were stained with primary antibodies, α-actinin (Sigma-Alrich, MO, USA), cardiac troponin T (USBiologicals, MA, USA), titin (DHSB, Iowa, USA), SERCA (Sigma-Alrich, MO, USA), MLC2v (Synaptic Systems, Germany), MLC2a (Synaptic Systems, Germany), Nkx2.5 (Abcam, MA, USA) and whole EBs with T, Mesp1 (Abcam, MA, USA) and cTnT and NKx2.5. Samples were washed and incubated with respective secondary antibodies (Invitrogen, CA, USA) for 1 hour and subsequently counterstained with DAPI. Slides were examined under Zeiss LSM710 NLO multi-photon confocal microscope (Carl Zeiss Inc, USA).

### Flow cytometry

Human iPS-derived CMs or day 8 EBs were collected and trypsinized with TrypLE (Invitrogen, CA, USA) for 5-7 min to obtain single cell suspension. Cells were washed with PBS and fixed using fix and perm cell permeabilization reagent (Invitrogen, CA, USA), except for Sirpa staining and incubated with either one of the antibodies, Sirpa conjugated-PE (BD Biosciences), Nkx2.5 (Novus) and cardiac troponin T (USBiologicals, MA, USA) for 1 h followed by incubation with appropriate Alexa flour 488 or 350 conjugated secondary antibody (Invitrogen, CA, USA). Samples were then acquired on FACS Aria II (BD Biosciences, CA, USA) and the data were analyzed utilising FlowJo ver 6.4 software. A total of 10,000 gated events were counted for each marker in three independent experiments.

### Real-time PCR

For real-time reverse-transcription polymerase chain reaction (qRT-PCR) analysis, RNA was isolated with the RNeasy kit (Qiagen GmbH, Hilden, Germany). One µg of total RNA was converted to complementary DNA by Superscript III first-strand synthesis system (Invitrogen, CA, USA). cDNA template (5 ng) was used from each sample and SYBR green real-time PCR studies were performed using Quantifast kit (Qiagen GmbH, Hilden, Germany) and primer (Supplementary table 1) as per the kit instructions. Samples were cycled with RotorGene Q (Qiagen GmbH, Hilden, Germany) as follows: 5 minutes at 95°C, followed by 40 cycles of 10 seconds at 95°C and 30 second extension at 60°C. All experiments were performed in triplicates. Relative quantification was calculated according to the ΔΔCt method for quantitative real-time PCR (using an endogenous control gene, GAPDH). For each gene, the expression at a specific day was then normalized by its baseline values. Heatmaps were generated from Ct values using Genesis software.

### Statistical analysis

Results were reported as mean ± standard error of mean (SEM). Comparison between groups was performed using One-way ANOVA followed by Tukey's post-hoc tests. p<0.05 was considered statistically significant.

### Results

### Standardization of multiple components

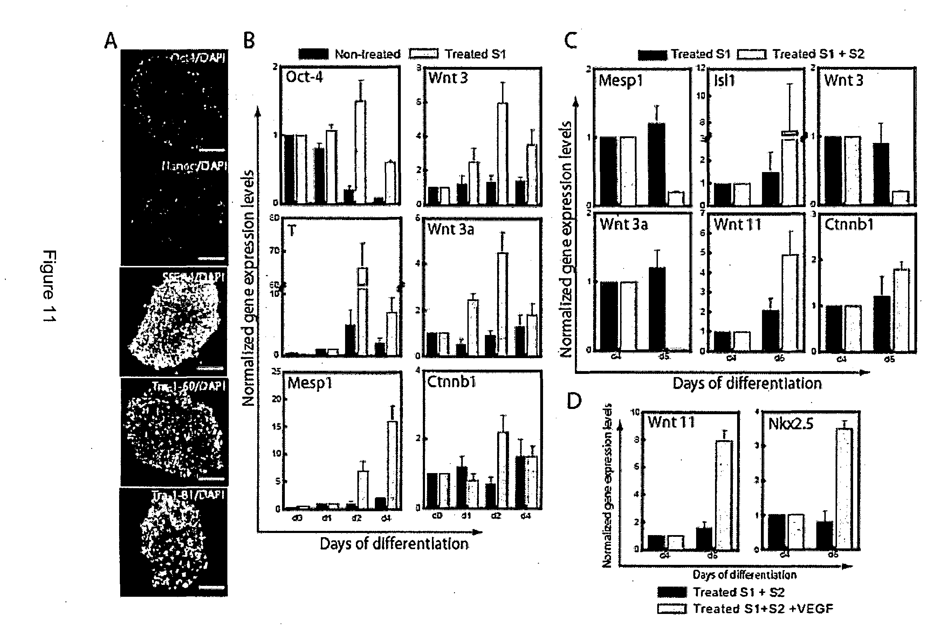

All pluripotent stem cell lines (hPSC) were maintained as feeder-free cultures. Colony morphology of these iPSCs was similar to hESC colonies in size and in nucleus/cytoplasmic ratio. Immunofluorescence staining showed strong nuclear staining for Oct-4 (Fig 11A) and various surface embryonic antigens (Nanog, SSEA4, Tra-1-60 and Tra-1-81) (Fig 11A), confirming their pluripotency.

To maintain consistency in differentiation efficiency, an EB based protocol was utilised to recapitulate early cardiac development that hinges on stage-specific activation and inhibition of Wnt/β-catenin along with BMP modulation. All initial experiments were performed using H3-Nkx2.5-GFP line to standardize the protocol. Spin EBs of different sizes (1,000, 2,500, 5,000, 7,500 and 10,000 cells/EB) were made and it was observed that EBs of 2,500 to 7,500 cells gave contracting cardiomyocytes by day 14-15 using only 10uM SB203580 (Figure 11B; Non-treated), with highest efficiencies with 5,000 cell EBs. Next. BMP4 and Activin A (Figure 11B; Treated S1) were incorporated to commit EBs towards mesoderm and cardiac fate. Gene expression studies during the initial stage of differentiation showed that this combination was sufficient to induce Brachyury at day 2 followed by Mesp1 up-regulation by day 4 (Figure 11B). It was also observed that combination of BMP/Activin A, increased expression of pluripotency markers Oct4 along with Wnt 3, Wnt 3a and CTNNB1 following treatment (Figure 11B).

Next, specific inhibitors, Noggin (BMP blocker), A83-01 (Activin/Nodal blocker) and IWP4 (antagonist of the Wnt/β-catenin pathway) were used for the next 2 days (Treated S1+S2). Gene expression analysis following addition of these blockers, demonstrated a significant down-regulation of Wnt3 and Wnt 3a, but maintained beta-catenin/CTNNB1, followed by an up-regulation of Wnt11 and Isl1 expression with corresponding decreased Mesp1 expression levels (Figure 11C). We next added VEGF (Figure 11 D; Treated S1+S2+VEGF) to promote proliferation of cardiac progenitors and addition of VEGF substantially increased gene expression levels of Wnt11 1 and activation of cardiac specific transcriptional factors like Nkx2.5 (Figure 11D). It was observed that there was noticeable cell death during the first 4 days of differentiation, hence ascorbic acid was incorporated in the medium throughout to promote cell survival.

### Cardiac differentiation across multiple lines and EB morphology

Upon standardisation, multiple pluripotent stem cell lines (hPSC) were subjected to cardiac differentiation via EB formation as shown in the schematically in Figure 12A. During the course of differentiation, all cell lines showed a significant increase in EB size from day 0 (range 0.02-0.04 µm², n=100) to day 8 (range 0.12-0.17 µm², n=50) (Figure 12B-C). While the hESC line (H3G) showed the best proliferation rates (d1 vs d8: 0.04±0.002 mm² vs 0.18± 0.008 mm²), hiPSC line AT and BJ had the least (d1 vs d8: AT, 0.02±0.002 mm² vs 0.13± 0.008 mm²; BJ, 0.03±0.002 mm² vs 0.12± 0.007 mm²) (Figure 2C). Nevertheless, all EBs generated from various cell lines demonstrated more than 80% contraction efficiency on day 15 of differentiation, with H3G, AT and BJ at 90% (Figure 12D, n=5). These observations clearly indicated that this cardiac differentiation protocol efficiently differentiated all 8 hPSC lines towards cardiac lineage.

### Temporal expression during cardiac commitment

Next, a time dependent (d0, 1, 2, 4, 6, 8, 14, 18) real-time gene expression profile of 35 established genes was performed to evaluate the cardiogenic trends during differentiation from 5 PSC lines (1 hESC and 4 hiPSC). A cumulative heatmap of these markers clearly showed that all lines followed a similar trend during cardiac differentiation to generate myocytes efficiently (Figure 13A). Consistent with prior optimization results, this extended real-time PCR analysis demonstrated a gradual, but significant decrease of pluripotency markers (Oct4, Sox2 and Nanog), which was accompanied with a spike up-regulation of T, mesodermal commitment (Figure 13B). Interestingly, this up-regulation was prominent in some lines on day 2 of differentiation (H3G, and C3), but on day 4 for others (BJ, K1 and AT) though their relative expression levels were varied (Figure 13B). Interestingly, all cell lines showed high levels of Mesp1, a cardiac commitment marker, on day 4 of differentiation (Figure 13B). Whole-mount immunostaining of day 4 EBs showed significant expression of T and Mesp1 throughout the EBs (Figure 13C). These results confirm that this differentiation strategy robustly differentiated multiple hiPSC towards cardiac commitment.

### Temporal expression during progenitor commitment

Significant increase of Mesp1 by day 4, was followed with expression of cardiac-associated transcriptional factors, NKx2.5 and Irx4 by day 8 (Figure 14A). Immunostaining of day 8 EBs showed high expression of Nkx2.5 (red) throughout with weak localized cTnT (<10%; green) staining (Figure 14B). Moreover, flow cytometric analysis was performed on day 8 EBs showed that >90% of the across all cell lines were positive for SIRPA as well as NKx2.5 (Figure 14C) with low levels of cTnT⁺ cells (>7%), except BJ cells (33.4 ± 4.74 %, Figure 14C). These results show that this differentiation cocktail significantly promoted cardiac commitment over a period of 8 days in human embryonic stem cells (hESC H3G), induced pluripotent stem cells of different origins and clinical status (healthy neonate [C3], healthy adult [AT], cardiac ischemia patient [14], LQTS2 patient [K1.3/K1.4], healthy neonate [BJ; reprogrammed by mRNA technique] and adult [8⁺/10⁻; reprogrammed by viral technique]).

### Temporal expression in cardiomyocytes

Day 8 EBs were plated and contracting area were visible across all the cell lines by day 13. Interestingly, in cell lines like H3G and BJ, contractions were evident as early as day 9-10. Concomitant with the expression of early cardiac transcriptional factors, there was a significant up regulation in the expression patterns of cardiac-specific structural and sarcomeric proteins (myosin light chain 2v, MLC2v; cardiac troponin T, cTnT; cardiac pacemaker channel, HCN4) till day 18 of differentiation (Figure 15A). In order to validate our qPCR data, immunostaining and flow cytometric analysis was performed. Dual staining on day 14 cardiomyocytes showed that majority of cells were positive for NKx2.5 (red, No. of nuclei, Nkx2.5⁺/DAPI⁺: 652/670) and a significant increase in cTnT⁺ (green, No of cTnT⁺/DAPI⁺: 358/670). Moreover, human iPSC-CMs on day 20 also stained positive for the sarcomeric proteins, cardiac actinin, titin, myosin light chain 2v and 2a and SERCA2a (Figure 5B). Positively stained cardiomyocytes demonstrated an immature striated pattern indicating towards early stages of myocyte development. However, Z-bands and A-bands were clearly visible in these cardiomyocytes. Flow cytometric analysis on day 18 dissociated cells demonstrated varying extent of cTnT⁺ cells, ranging from 55-95% (Figure 15C). It was interesting to note that while H3G, BJ and AT gave the highest percentage of cTnT⁺Nkx2.5⁺ (>90%), C3 lines showed relatively lower efficiency (55.7±4.5 %, n=7) in comparison with the other lines (Figure 15C). These results confirm that our protocol effectively differentiated multiple pluripotent stem cells to cardiomyocytes with high consistency.

### Role of WNTs in cardiac differentiation

Next, a total of 15 WNT members was evaluated to understand their role during cardiac differentiation. These gene expression results indicated that Wnt 2/2b, 3/3a, 5a/5b and Wnt 11, played critical roles in regulating cardiogenesis during various stage of differentiation (Figure 6A). Wnt 3/3a, a member of the canonical pathway, was significantly up regulated in all 5 cell lines (H3G, C3, K1, AT and BJ) over a period of day 1-4 (Figure 16B) and their expression was significantly reduced post-day 4, except for cell line C3 where it reappeared on day 8, On the other hand, Wnt 11, a non-canonical regulator, was significantly up-regulated post day 6 to 8 in all the cell lines and was the highest on day 18 of differentiation (Figure 16B). These results clearly indicate that the medium components regulated the Wnt signaling cascade by first activating the canonical pathway for early cardiac commitment and then non-canonical pathway for cardiac specification.

### Discussion

Cardiogenesis is a well-organized process that is tightly regulated by signaling factors and extracellular microenvironment. Although cardiomyocytes have been successfully generated from mouse as well as human PSC, *in vitro,* intra-and inter-line efficiency of hPSCs have been highly variable.

This study demonstrated that by systematically optimising the inductive pathway, it was possible to recapitulate early mammalian cardiac development that significantly improved cardiomyogeneis across multiple PSC lines without extensive titration of cytokines involved. One of the key features during EB differentiation is regulating the size of the EB, it was observed that size of EBs (5,000 cells EBs) is highly critical in efficient cardiac differentiation across multiple lines. Furthermore, using high throughput microwell could significantly enhance the large-scale production of EBs during each differentiation (n= 2,400 EBs per plate).

Differentiation from pluripotent stem cells towards cardiomyocytes could be divided into 4 stages; mesodermal induction, patterning towards cardiac mesoderm, formation of cardiac mesoderm and early cardiomyocytes. BMP4 is a potent mesodermal inducer, however it alone is insufficient to regulate the developmental process. Mesodermal commitment is highly dependent on interactive actions of BMP/Nodal/Wnt signaling pathways. Simultaneous addition of Activin A (an inducer for endoderm) with BMP4 not only enhanced CTNNB1/β-catenin levels in hPSC differentiation, but also increased expression of canonical Wnts (Wnt3 and 3a) along with brachyury. Immunostaining of day 4 EBs post BMP4/Activin A treatment showed significant expression of T and Mesp1 confirming successful mesodermal commitment and patterning towards cardiac mesoderm.

Mesp1 is a master regulator of cardiovascular development and has been suggested to initiating cardiac transcription factor cascade for cardiac development. Our results showed a marked enhancement in cardiac gene expression levels post-Mesp1 up regulation. However, this up-regulation coincided with significant down-regulation of canonical Wnts. This is consistent with the requirement of Wnt/CTNNB1 signalling restriction during specification of cardiac precursor cells.

In this study, it was observed that the examined cell lines showed marked differences in the canonical Wnt levels during first four days of differentiation. It is hypothesized that post-mesodermal commitment, down-regulation of canonical Wnts may be critical for converting mesodermal cells towards cardiac fate. This could explain the great variability and inconsistency observed in current available cardiac differentiation protocols in the literature. To confirm this hypothesis, on post-day 4 of differentiation (after mesodermal commitment), added Wnt inhibitor, IWP4 and Activin/Nodal inhibitor A83-01 and BMP inhibitor, Noggin was added. This significantly reduced levels of canonical Wnts, thereby pushing Mesp1+ cells towards cardiac commitment. Interestingly, this combination not only, reduced canonical Wnt levels, but also significantly increased non-canonical Wnts (Wnt 11 and 5a). Consistently, loss-of-function and overexpression experiments have shown that Wnt 11 is critical for normal heart development and promotes cardiac activity.

The flow cytomertic analysis show that all cell lines demonstrate more than 75% of troponin positive cells, except C3 (56%). From the present detailed protocol, for every 1.5 million initiating pluripotent stem cells, an average of 2 to 2.5 million cardiomyocytes could be efficiently generated across multiple cell lines.

In summary, the present protocol yields cardiomyocytes with high consistency and purity from a wide variety of normal or patient derived hiPSC lines created through viral, episomal or mRNA based techniques. More importantly, it was demonstrated that the differentiation strategy recapitulates cardiac development by timely regulating the Wnt signalling pathway resulting in efficient generation of large quantities of cardiomyocytes. The present protocol enables a universal cardiac platform for high throughput drug screening, safety pharmacology evaluation and regenerative therapy that demand consistent cost-effectiveness in cardiomyocyte production.

### References

Graichen et al., (2008) Enhanced cardiomyogenesis of human embryonic stem cells by a small molecular inhibitor of p38 MAPK. Differentiation (2008) 76:357-370.

Hudson et al., (2012) Primtive cardiac cells from human embryonic stem cells. Stem cells and development 21 (9):1513-1523.

Mehta et al., (2011) Pharmacological response of human cardiomyocytes derived from virus-free induced pluripotent stem cells. Cardiovascular research 91 (4):577-86.

Moore et al., (2008) Distinct cardiogenic preferences of two human embryonic stem cell (hESC) lines are imprinted in their proteomes in the pluripotent state. Biochemical and biophysical research communications 372(4): 553-8.

Sachinidis et al., (2006) Identification of small signalling molecles promoting cardiac-specific differentiation of mouse embryonic stem cells. Cellular Physiology and Biochemistry 18:303-314.

## Claims

1. An *in vitro* method for inducing cardiomyocyte differentiation comprising the steps of:
(i) providing at least one induced pluripotent stem cell or embryoid body comprising induced pluripotent stem cells;
(ii) contacting said pluripotent stem cell(s) or said embryoid body with:
(a) SB 203580 (4-[4-(4-fluorophenyl)-2-(4-methylsulfinylphenyl)-1H-imidazol-5-yl]pyridine;
(b) cyclosporin-A (CSA); and
(c) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) at a final concentration of 5 µM.

2. The method according to claim 1, comprising contacting said pluripotent stem cell(s) or said embryoid body with SB 203580 (4-[4-(4-fluorophenyl)-2-(4-methylsulfinylphenyl)-1H-imidazol-5-yl]pyridine; cyclosporin-A (CSA) and said final concentration of IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) sequentially, in any order.

3. The method according to claim 1 or 2, comprising contacting said pluripotent stem cell(s) or said embryoid body with SB 203580 (4-[4-(4-fluorophenyl)-2-(4-methylsulfinylphenyl)-1H-imidazol-5-yl]pyridine; cyclosporin-A (CSA) and said final concentration of IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) in the order listed.

4. The method according to claim 1, comprising contacting said pluripotent stem cell(s) or said embryoid body with SB 203580 (4-[4-(4-fluorophenyl)-2-(4-methylsulfinylphenyl)-1H-imidazol-5-yl]pyridine, cyclosporin-A (CSA) and said final concentration of IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) concurrently.

5. The method according to claim 4, comprising contacting said pluripotent stem cell(s) or said embryoid body with a composition comprising SB 203580 (4-[4-(4-fluorophenyl)-2-(4-methylsulfinylphenyl)-1H-imidazol-5-yl]pyridine, cyclosporin-A (CSA) and IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) at said final concentration.

6. The method according to any one of the preceding claims, wherein said pluripotent stem cell(s) comprises isolated pluripotent stem cell(s).

7. The method according to any one of the preceding claims, comprising the steps of:
(i) inducing said pluripotent stem cells to form at least one embryoid body;
(ii) contacting the embryoid body with:
(a) SB 203580 (4-[4-(4-fluorophenyl)-2-(4-methylsulfinylphenyl)-1H-imidazol-5-yl]pyridine;
(b) cyclosporin-A (CSA) and
(c) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) at said final concentration.

8. The method according to claim 7, wherein step (ii) comprises contacting said embryoid body with:
(i) SB 203580 4-[4-(4-fluorophenyl)-2-(4-methylsulfinylphenyl)-1H-imidazol-5-yl]pyridine at a final concentration of 5 µM from day 2 to day 8 post embryoid body induction;
(ii) cyclosporin-A (CSA) at a final concentration of 3µg/ml from day 4 to day 8 post embryoid body induction; and
(iii) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) at a final concentration of 5 µM from day 4 to day 8 post embryoid body induction.

9. The method according to any one of the preceding claims , comprising
(i) contacting said pluripotent stem cell(s) or said embryoid body with a composition B comprising SB 203580 (4-[4-(4-fluorophenyl)-2-(4-methylsulfinylphenyl)-1H-imidazol-5-yl]pyridine,
(ii) contacting said pluripotent stem cell(s) or said embryoid body with a composition C comprising SB 203580 (4-[4-(4-fluorophenyl)-2-(4-methylsulfinylphenyl)-1H-imidazol-5-yl]pyridine, cyclosporin-A (CSA) and IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) to give said final concentration of IWP4 and
(iii) contacting said pluripotent stem cell(s) or said body with a composition D comprising SB 203580 (4-[4-(4-fluorophenyl)-2-(4-methylsulfinylphenyl)-1H-imidazol-5-yl]pyridine, cyclosporin-A (CSA) and IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamide) to give said final concentration of IWP4.

10. The method according to claim 9, wherein composition B further comprises ascorbic acid, bone morphogenic protein 4 (BMP4), activin A and fibroblast growth factor 2 (FGF2).

11. The method according to claim 9 or 10, wherein composition C further comprises ascorbic acid, Noggin, A83-01 and vascular endothelial growth factor (VEGF).

12. The method according to any one of claims 9 to 11, wherein composition D further comprises ascorbic acid and vascular endothelial growth factor (VEGF).

13. The method according to any one of claims 10 to 12, wherein composition C further comprises the epidermal growth factor receptor kinase inhibitor AG1478.

14. The method according to any one of claims 10 to 13, wherein composition D further comprises the epidermal growth factor receptor kinase inhibitor AG1478.

15. The method according to any one of the preceding claims, further comprising contacting said pluripotent stem cell(s) or said embryoid body with H₂O₂.

16. The method according to any one of the preceding claims, wherein said pluripotent stem cells or said embryoid body are induced to differentiate into cardiomyocytes via monolayer differentiation.

## Patentansprüche

1. *In vitro* Verfahren zum Induzieren von Cardiomyocytendifferenzierung umfassend die Schritte:
(i) Bereitstellen von wenigstens einer pluripotenten Stammzelle oder eines embryoartigen Körpers umfassend pluripotente Stammzellen;
(ii) Kontaktieren der pluripotenten Stammzelle(n) oder des embryoartigen Körpers mit
(a) SB 203580 (4-[4-(4-Fluorphenyl)-2-(4-methylsulfinylphenyl)-1H-imidazol-5-yl]pridin);
(b) Cyclosporin-A (CSA); und
(c) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid) mit einer Endkonzentration von 5 µM.

2. Verfahren nach Anspruch 1, umfassend sequenzielles Kontaktieren der pluripotenten Stammzelle(n) oder des embryoartigen Körpers, in einer jeglichen Reihenfolge, mit SB 203580 (4-[4-(4-Fluorphenyl)-2-(4-methylsulfinylphenyl)-1H-imidazol-5-yl]pridin); Cyclosporin-A (CSA) und der Endkonzentration von IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid).

3. Verfahren nach Anspruch 1 oder 2, umfassend Kontaktieren der pluripotenten Stammzelle(n) oder des embryoartigen Körpers mit SB 203580 (4-[4-(4-Fluorphenyl)-2-(4-methylsulfinylphenyl)-1H-imidazol-5-yl]pridin); Cyclosporin-A (CSA) und der Endkonzentration von IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid) in der angegebenen Reihenfolge.

4. Verfahren nach Anspruch 1, umfassend gleichzeitiges Kontaktieren der pluripotenten Stammzelle(n) oder des embryoartigen Körpers mit SB 203580 (4-[4-(4-Fluorphenyl)-2-(4-methylsulfinylphenyl)-1H-imidazol-5-yl]pridin); Cyclosporin-A (CSA) und der Endkonzentration von IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid).

5. Verfahren nach Anspruch 4, umfassend Kontaktieren der pluripotenten Stammzelle(n) oder des embryoartigen Körpers mit einer Zusammensetzung umfassend SB 203580 (4-[4-(4-Fluorphenyl)-2-(4-methylsulfinylphenyl)-1H-imidazol-5-yl]pridin); Cyclosporin-A (CSA) und IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid) mit der Endkonzentration.

6. Verfahren nach einem der vorangehenden Ansprüche, wobei die pluripotente(n) Stammzelle(n) isolierte pluripotente Stammzelle(n) umfasst/umfassen.

7. Verfahren nach einem der vorangehenden Ansprüche, umfassend die Schritte:
(i) Induzieren der pluripotenten Stammzellen, so dass sie wenigstens einen embryoartigen Körper ausbilden;
(ii) Kontaktieren des embryoartigen Körpers mit:
(a) SB 203580 (4-[4-(4-Fluorphenyl)-2-(4-methylsulfinylphenyl)-1H-imidazol-5-yl]pridin);
(b) Cyclosporin-A (CSA); und
(c) der Endkonzentration von IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid).

8. Verfahren nach Anspruch 7, wobei Schritt (ii) umfasst Kontaktieren des embryoartigen Körpers mit
(i) SB 203580 (4-[4-(4-fluorphenyl)-2-(4-methylsulfinylphenyl)-1H-imidazol-5-yl]pridin) mit einer Endkonzentration von 5 µM von Tag 2 bis Tag 8 nach Induktion des embryoartigen Körpers;
(ii) Cyclosporin-A (CSA) mit einer Endkonzentration von 3 µg/ml von Tag 4 bis Tag 8 nach Induktion des embryoartigen Körpers; und
(iii) IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid) mit einer Endkonzentration von 5 µM von Tag 4 bis Tag 8 nach Induktion des embryoartigen Körpers.

9. Verfahren nach einem der vorangehenden Ansprüche, umfassend
(i) Kontaktieren der pluripotenten Stammzelle(n) oder des embryoartigen Körpers mit einer Zusammensetzung B umfassend SB 203580 (4-[4-(4-Fluorphenyl)-2-(4-methylsulfinylphenyl)-1H-imidazol-5-yl]pridin),
(ii) Kontaktieren der pluripotenten Stammzelle(n) oder des embryoartigen Körpers mit einer Zusammensetzung C umfassend SB 203580 (4-[4-(4-Fluorphenyl)-2-(4-methylsulfinylphenyl)-1H-imidazol-5-yl]pridin), Cyclosporing-A (CSA) und IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid), um die Endkonzentration von IWP4 zu erhalten; und
(iii) Kontaktieren der pluripotenten Stammzelle(n) oder des Körpers mit einer Zusammensetzung D umfassend SB 203580 (4-[4-(4-Fluorphenyl)-2-(4-methylsulfinylphenyl)-1H-imidazol-5-yl]pridin, Cyclosporing-A (CSA) und IWP4 (2-(3,4,6,7-tetrahydro-3-(2-methoxyphenyl)-4-oxothieno[3,2-d]pyrimidin-2-ylthio)-N-(6-methylbenzo[d]thiazol-2-yl)acetamid), um die Endkonzentration von IWP4 zu erhalten.

10. Verfahren nach Anspruch 9, wobei Zusammensetzung B weiter Ascorbinsäure, knochenbildendes Protein 4 (BMP4), Activin A und Fibroblastenwachstumsfaktor 2 (FGF2) umfasst.

11. Verfahren nach Anspruch 9 oder 10, wobei Zusammensetzung C weiter Ascorbinsäure, Noggin, A83-01 und vaskulären endothelialen Wachstumsfaktor (VEGF) umfasst.

12. Verfahren nach einem der Ansprüche 9 bis 11, wobei Zusammensetzung D weiter Ascorbinsäure und vaskulären endothelialen Wachstumsfaktor (VEGF) umfasst.

13. Verfahren nach einem der Ansprüche 10 bis 12, wobei Zusammensetzung C weiter den Inhibitor der epidermalen Wachstumsfaktor-Rezeptorkinase AG1478 umfasst.

14. Verfahren nach einem der Ansprüche 10 bis 13, wobei Zusammensetzung D weiter den Inhibitor der epidermalen Wachstumsfaktor-Rezeptorkinase AG1478 umfasst.

15. Verfahren nach einem der vorangehenden Ansprüche, weiter umfassend Kontaktieren der pluripotenten Stammzelle(n) oder des embryoartigen Körpers mit H₂O₂.

16. Verfahren nach einem der vorangehenden Ansprüche, wobei die pluripotenten Stammzellen oder der embryoartige Körper induziert werden, um durch Monolayer-Differenzierung zu Cardiomyocyten zu differenzieren.

## Revendications

1. Procédé *in vitro* pour induire une différenciation en cardiomyocytes comprenant les étapes qui consistent :
(i) à fournir au moins une cellule souche pluripotente induite ou un corps embryoïde comprenant des cellules souches pluripotentes induites ;
(ii) à mettre en contact ladite/lesdites cellule(s) souche(s) pluripotente(s) ou ledit corps embryoïde avec :
(a) SB 203580 (4-[4-(4-fluorophényl)-2-(4-méthylsulfinylphényl)-1H-imidazol-5-yl]pyridine) ;
(b) la cyclosporine-A (CSA) ; et
(c) IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) à une concentration finale de 5 µM.

2. Procédé selon la revendication 1, comprenant le fait de mettre en contact ladite/lesdites cellule(s) souche(s) pluripotente(s) ou ledit corps embryoïde avec SB 203580 (4-[4-(4-fluorophényl)-2-(4-méthylsulfinylphényl)-1H-imidazol-5-yl]pyridine) ; la cyclosporine-A (CSA) et ladite concentration finale de IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) de manière séquentielle, dans n'importe quel ordre.

3. Procédé selon la revendication 1 ou 2, comprenant le fait de mettre en contact ladite/lesdites cellule(s) souche(s) pluripotente(s) ou ledit corps embryoïde avec SB 203580 (4-[4-(4-fluorophényl)-2-(4-méthylsulfinylphényl)-1H-imidazol-5-yl]pyridine) ; la cyclosporine-A (CSA) et ladite concentration finale de IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) dans l'ordre indiqué.

4. Procédé selon la revendication 1, comprenant le fait de mettre en contact ladite/lesdites cellule(s) souche(s) pluripotente(s) ou ledit corps embryoïde avec SB 203580 (4-[4-(4-fluorophényl)-2-(4-méthylsulfinylphényl)-1H-imidazol-5-yl]pyridine) ; la cyclosporine-A (CSA) et ladite concentration finale de IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) simultanément.

5. Procédé selon la revendication 4, comprenant le fait de mettre en contact ladite/lesdites cellule(s) souche(s) pluripotente(s) ou ledit corps embryoïde avec une composition comprenant SB 203580 (4-[4-(4-fluorophényl)-2-(4-méthylsulfinylphényl)-1H-imidazol-5-yl]pyridine), la cyclosporine-A (CSA) et IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) à ladite concentration finale.

6. Procédé selon l'une quelconque des revendications précédentes, dans lequel ladite/lesdites cellule(s) souche(s) pluripotente(s) comprend/comprennent une/des cellule(s) souche(s) pluripotente(s) isolée(s).

7. Procédé selon l'une quelconque des revendications précédentes, comprenant les étapes qui consistent :
(i) à induire lesdites cellules souches pluripotentes pour former au moins un corps embryoïde ;
(ii) à mettre en contact le corps embryoïde avec :
(a) SB 203580 (4-[4-(4-fluorophényl)-2-(4-méthylsulfinylphényl)-1H-imidazol-5-yl]pyridine) ;
(b) la cyclosporine-A (CSA) ; et
(c) IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) à ladite concentration finale.

8. Procédé selon la revendication 7, dans lequel l'étape (ii) comprend le fait de mettre en contact ledit corps embryoïde avec :
(i) SB 203580 4-[4-(4-fluorophényl)-2-(4-méthylsulfinylphényl)-1H-imidazol-5-yl]pyridine à une concentration finale de 5 µM du jour 2 au jour 8 après l'induction du corps embryoïde ;
(ii) la cyclosporine-A (CSA) à une concentration finale de 3µg/ml du jour 4 au jour 8 après l'induction du corps embryoïde ; et
(iii) IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) à une concentration finale de 5 µM du jour 4 au jour 8 après l'induction du corps embryoïde.

9. Procédé selon l'une quelconque des revendications précédentes, comprenant le fait
(i) de mettre en contact ladite/lesdites cellule(s) souche(s) pluripotente(s) ou ledit corps embryoïde avec une composition B comprenant SB 203580 (4-[4-(4-fluorophényl)-2-(4-méthylsulfinylphényl)-1H-imidazol-5-yl]pyridine),
(ii) de mettre en contact ladite/lesdites cellule(s) souche(s) pluripotente(s) ou ledit corps embryoïde avec une composition C comprenant SB 203580 (4-[4-(4-fluorophényl)-2-(4-méthylsulfinylphényl)-1H-imidazol-5-yl]pyridine), la cyclosporine-A (CSA) et IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) pour donner ladite concentration finale de IWP4 et
(iii) de mettre en contact ladite/lesdites cellule(s) souche(s) pluripotente(s) ou ledit corps avec une composition D comprenant SB 203580 (4-[4-(4-fluorophényl)-2-(4-méthylsulfinylphényl)-1H-imidazol-5-yl]pyridine), la cyclosporine-A (CSA) et IWP4 (2-(3,4,6,7-tétrahydro-3-(2-méthoxyphényl)-4-oxothiéno[3,2-d]pyrimidin-2-ylthio)-N-(6-méthylbenzo[d]thiazol-2-yl)acétamide) pour donner ladite concentration finale de IWP4.

10. Procédé selon la revendication 9, dans lequel la composition B comprend en outre l'acide ascorbique, la protéine morphogénétique osseuse 4 (BMP4), l'activine A et le facteur de croissance des fibroblastes 2 (FGF2).

11. Procédé selon la revendication 9 ou 10, dans lequel la composition C comprend en outre l'acide ascorbique, la Noggin, A83-01 et le facteur de croissance de l'endothélium vasculaire (VEGF).

12. Procédé selon l'une quelconque des revendications 9 à 11, dans lequel la composition D comprend en outre l'acide ascorbique et le facteur de croissance de l'endothélium vasculaire (VEGF).

13. Procédé selon l'une quelconque des revendications 10 à 12, dans lequel la composition C comprend en outre l'inhibiteur de kinase du récepteur du facteur de croissance épidermique AG1478.

14. Procédé selon l'une quelconque des revendications 10 à 13, dans lequel la composition D comprend en outre l'inhibiteur de kinase du récepteur du facteur de croissance épidermique AG1478.

15. Procédé selon l'une quelconque des revendications précédentes, comprenant en outre le fait de mettre en contact ladite/lesdites cellule(s) souche(s) pluripotente(s) ou ledit corps embryoïde avec H₂O₂.

16. Procédé selon l'une quelconque des revendications précédentes, dans lequel lesdites cellules souches pluripotentes ou ledit corps embryoïde sont/est induit(es) pour se différencier en cardiomyocytes par une différenciation monocouche.


### SEQUENCE LISTING

<110> Singapore Health Services Pte Ltd
   Shim, winston se Ngie
   Mehta, Ashish
   Sequeira, Glen Lester
<120> Method, comibnation and/or composition for inducing cardiomyocyte differentiation
<130> FP6607
<150> US 61/680512
   <151> 2012-08-07
<160> 38
<170> PatentIn version 3.5
<210> 1
   <211> 20
   <212> DNA
   <213> Artificial
<220>
   <223> GAPDH forward primer
<400> 1
   gtggacctga cctgccgtct 20
<210> 2
   <211> 20
   <212> DNA
   <213> artificial
<220>
   <223> GAPDH reverse primer
<400> 2
   ggaggagtgg gtgtcgctgt 20
<210> 3
   <211> 20
   <212> DNA
   <213> artificial
<220>
   <223> OCT-4 forward primer
<400> 3
   agtttgtgcc agggtttttg 20
<210> 4
   <211> 20
   <212> DNA
   <213> artificial
<220>
   <223> OCT-4 reverse primer
<400> 4
   acttcacctt ccctccaacc 20
<210> 5
   <211> 20
   <212> DNA
   <213> artificial
<220>
   <223> GATA-4 forward primer
<400> 5
   tccaaaccag aaaacggaag 20
<210> 6
   <211> 20
   <212> DNA
   <213> artificial
<220>
   <223> CATA-4 reverse primer
<400> 6
   aaggctctca ctgcctgaag 20
<210> 7
   <211> 20
   <212> DNA
   <213> artificial
<220>
   <223> ISL-1 forward primer
<400> 7
   aaggacaaga agagaagcat 20
<210> 8
   <211> 20
   <212> DNA
   <213> artificial
<220>
   <223> ISL-1 reverse primer
<400> 8
   catgggagtt cctgtcatcc 20
<210> 9
   <211> 18
   <212> DNA
   <213> Brachyury (T) forward primer
<400> 9
   tcggggccca ctggatga 18
<210> 10
   <211> 18
   <212> DNA
   <213> artificial
<220>
   <223> Brachyury (T) reverse primer
<400> 10
   atgcgctgtg gaccccca 18
<210> 11
   <211> 20
   <212> DNA
   <213> artificial
<220>
   <223> HAND1 forward primer
<400> 11
   aagctttccc tgtgttggaa 20
<210> 12
   <211> 20
   <212> DNA
   <213> artificial
<220>
   <223> HAND1 reverse primer
<400> 12
   ggcaggatga acaaacacct 20
<210> 13
   <211> 18
   <212> DNA
   <213> artificial
<220>
   <223> MESP1 forward primer
<400> 13
   ggcagaggca gagcgcca 18
<210> 14
   <211> 19
   <212> DNA
   <213> artificial
<220>
   <223> MESP1 reverse primer
<400> 14
   agcggatagc caggcgcag 19
<210> 15
   <211> 21
   <212> DNA
   <213> artificial
<220>
   <223> MEF-2C forward primer
<400> 15
   cccaacctat tgccactggct 21
<210> 16
   <211> 21
   <212> DNA
   <213> artificial
<220>
   <223> MEF-2C reverse primer
<400> 16
   atacccgttc cctgcactgg t 21
<210> 17
   <211> 20
   <212> DNA
   <213> artificial
<220>
   <223> NKX 2.5 forward primer
<400> 17
   ctaaacctgg aacagcagca 20
<210> 18
   <211> 20
   <212> DNA
   <213> artificial
<220>
   <223> NKX 2.5 reverse primer
<400> 18
   gtaggcctct ggcttgaagg 20
<210> 19
   <211> 21
   <212> DNA
   <213> artificial
<220>
   <223> TBX5 forward primer
<400> 19
   tctgtgacgg gcaaagctga g 21
<210> 20
   <211> 23
   <212> DNA
   <213> artificial
<220>
   <223> TBX5 reverse primer
<400> 20
   aatatgccca aatgggtcca ggt 23
<210> 21
   <211> 19
   <212> DNA
   <213> artificial
<220>
   <223> TBX20 forward primer
<400> 21
   agtggcagca gcccgtcct 19
<210> 22
   <211> 21
   <212> DNA
   <213> artificial
<220>
   <223> TBX20 reverse primer
<400> 22
   catctcggtg cccagctcat g 21
<210> 23
   <211> 23
   <212> DNA
   <213> artificial
<220>
   <223> CACNA1D forward primer
<400> 23
   gggcaatggg acctcataaa taa 23
<210> 24
   <211> 22
   <212> DNA
   <213> artificial
<220>
   <223> CACNA1D reverse primer
<400> 24
   ttacctggtt gcgagtgcat ta 22
<210> 25
   <211> 20
   <212> DNA
   <213> artificial
<220>
   <223> RYR2 forward primer
<400> 25
   caggaagtga ggcagcccaa 20
<210> 26
   <211> 22
   <212> DNA
   <213> artificial
<220>
   <223> RYR2 reverse primer
<400> 26
   cagacacagc gccaccttca ta 22
<210> 27
   <211> 20
   <212> DNA
   <213> artificial
<220>
   <223> HCN2 forward primer
<400> 27
   cacctgctac gccatgttca 20
<210> 28
   <211> 19
   <212> DNA
   <213> artificial
<220>
   <223> HCN2 reverse primer
<400> 28
   ctggcagctt gtggaagga 19
<210> 29
   <211> 20
   <212> DNA
   <213> artificial
<220>
   <223> KCNH2 forward primer
<400> 29
   ctgatcgggc tgctgaagac 20
<210> 30
   <211> 19
   <212> DNA
   <213> artificial
<220>
   <223> KCNH2 reverse primer
<400> 30
   agccaatgag catgacgca 19
<210> 31
   <211> 26
   <212> DNA
   <213> artificial
<220>
   <223> SERCA2 forward primer
<400> 31
   ccaaagtcat ctcccttatt tgcatt 26
<210> 32
   <211> 23
   <212> DNA
   <213> artificial
<220>
   <223> SERCA2 reverse primer
<400> 32
   gaccttcagg aatggctgct aca 23
<210> 33
   <211> 18
   <212> DNA
   <213> artificial
<220>
   <223> MLC2V forward primer
<400> 33
   ccttgggcga gtgaacgt 18
<210> 34
   <211> 20
   <212> DNA
   <213> artificial
<220>
   <223> MLC2V reverse primer
<400> 34
   gggtccgctc ccttaagttt 20
<210> 35
   <211> 21
   <212> DNA
   <213> artificial
<220>
   <223> MHY7 forward primer
<400> 35
   ggcaagacag tgaccgtgaa g 21
<210> 36
   <211> 22
   <212> DNA
   <213> artificial
<220>
   <223> MHY7 reverse primer
<400> 36
   cgtagcgatc cttgaggttg ta 22
<210> 37
   <211> 19
   <212> DNA
   <213> artificial
<220>
   <223> CTnI forward primer
<400> 37
   ccaactaccg cgcttatgc 19
<210> 38
   <211> 20
   <212> DNA
   <213> artificial
<220>
   <223> CTnI reverse primer
<400> 38
   ctcgctccag ctcttgcttt 20